# EUROPÄISCHE PATENTSCHRIFT

(11) **EP 3 727 291 B1**
(45) Veröffentlichungstag und Bekanntmachung des Hinweises auf die Patenterteilung: **07.05.2025**
(21) Anmeldenummer: 18806997.5
(22) Anmeldetag: 12.11.2018
(51) Int. Cl.: A45D 37/00, A61K 8/25, A45D 40/18, A61K 8/26, A61K 8/27, A61K 8/28, A61K 8/29, A61K 8/34, A61K 8/58, A61Q 5/10, A61K 8/81, A61K 8/85, A61K 8/02, B32B 27/08, B65D 81/32, B32B 7/12, B32B 27/32, B32B 27/36

(54) **WASSERSTOFFPEROXID-FORMULIERUNGEN IN SPERRSCHICHT-FOLIEN MIT EINER SIOX-SCHICHT**
HYDROGEN PEROXIDE FORMULATIONS IN BARRIER LAYER FILMS WITH A SIOX LAYER
FORMULATIONS DE PEROXYDE D'HYDROGENE DANS DES FILMS DE COUCHE BARRIERE AVEC UNE COUCHE DE SIOX

(30) Priorität: 18.12.2017 DE 102017223056
(43) Veröffentlichungstag der Anmeldung: 28.10.2020
(73) Patentinhaber: Henkel AG & Co. KGaA, 40589 Düsseldorf (DE)
(72) Erfinder: NOWOTTNY, Marc, 41069 Mönchengladbach (DE); LECHNER, Torsten, 40764 Langenfeld (DE); BARTHEL, Wolfgang, 40764 Langenfeld (DE)
(86) Internationale Anmeldenummer: PCT/EP2018/080864
(87) Internationale Veröffentlichungsnummer: WO 2019/120746

(56) Entgegenhaltungen:
- EP-A1- 0 792 846
- WO-A1-03/089330
- WO-A1-2016/096284
- DE-A1- 102010 029 206
- DE-A1- 102015 223 838

## Beschreibung

Die vorliegende Erfindung liegt auf dem Gebiet der Kosmetik und betrifft ein Produkt zur oxidativen Farbveränderung von keratinischen Fasern, insbesondere menschlichen Haaren, welches eine in einer Verpackung verpackte Oxidationsmittel-haltige Zusammensetzung umfasst. Die Oxidationsmittel-haltige Zusammensetzung enthält mindestens einen C₈-C₃₀-Alkohol und mindestens ein nichtionisches Tensid. Bei der Verpackung handelt es sich um eine aus einem speziellen mehrschichtigen Folien-Verbundsystem gefertigte Verpackung, deren Wandung mindestens zwei polymere Schichten und eine Barierre-Schicht umfasst. Hierbei besitzt die Barriere-Schicht eine Durchtrittsperrwirkung für Gase und Wasserdampf. Die Barriereschicht umfasst ein Siliciumoxid.

Die Veränderung der Farbe von keratinischen Fasern, insbesondere von Haaren, stellt einen wichtigen Bereich der modernen Kosmetik dar. Hierdurch kann das Erscheinungsbild der Haare sowohl aktuellen Modeströmungen als auch den individuellen Wünschen der einzelnen Person angepasst werden. Zur Farbveränderung der Haare kennt der Fachmann verschiedene Möglichkeiten. Durch den Einsatz von direktziehenden Farbstoffen kann die Haarfarbe temporär verändert werden. Hierbei diffundieren bereits fertig ausgebildete Farbstoffe aus dem Färbemittel in die Haarfaser hinein. Die Färbung mit direktziehenden Farbstoffen ist mit einer geringen Haarschädigung verbunden, ein Nachteil ist jedoch die geringe Haltbarkeit und die schnelle Auswaschbarkeit der mit direktziehenden Farbstoffen erhaltenen Färbungen.

Wünscht sich der Verbraucher ein lang anhaltendes Farbergebnis oder eine Nuance, die heller als seine Ausgangshaarfarbe ist, werden daher üblicherweise oxidative Farbveränderungsmittel eingesetzt. Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwickler-Komponenten und Kuppler-Komponenten, die unter dem Einfluss von Oxidationsmitteln - in der Regel Wasserstoffperoxid - untereinander die eigentlichen Farbstoffe ausbilden. Oxidationsfärbemittel zeichnen sich durch hervorragende, lang anhaltende Färbeergebnisse aus.

Oxidative Farbveränderungsmittel kommen üblicherweise in Form von Zweikomponenten-Mitteln auf den Markt, bei welchen zwei verschiedene Zubereitungen in zwei getrennten Verpackungen separat konfektioniert vorliegen und erst kurz vor der Anwendung miteinander vermischt werden. Bei der ersten Zubereitung handelt es sich um eine - aus Stabilitätsgründen in der Regel sauer eingestellte - Formulierung, welche als Oxidationsmittel beispielsweise Wasserstoffperoxid in Konzentrationen von 1,5 bis 12 Gew.-% enthält. Die Oxidationsmittelformulierung liegt meist in Form einer Emulsion oder Dispersion vor und wird in der Regel in einer Kunststoff-Flasche mit wiederverschließbarer Auslassöffnung zur Verfügung gestellt (Entwicklerflasche).

Diese Oxidationsmittel-Formulierung wird vor der Anwendung mit einer zweiten Zubereitung vermischt. Bei dieser zweiten Zubereitung handelt es sich um eine alkalisch eingestellte Formulierung, die oft in Form einer Creme oder eines Gels vorliegt und welche, sofern gleichzeitig mit der Aufhellung auch eine Farbänderung gewünscht wird, zusätzlich auch mindestens ein Oxidationsfarbstoffvorprodukt enthält. Diese zweite Zubereitung kann beispielsweise in Form einer Tube oder in Form eines Kunststoff- oder Glascontainers bereitgestellt werden.

Bei der zuvor beschriebenen üblichen Anwendungsform wird die zweite Zubereitung, welche das Alkalisierungsmittel und/oder die Oxidationsfarbstoffvorprodukte enthält, aus der Tube oder dem Container in die Entwicklerflasche überführt und dann durch Schütteln mit der bereits in der Entwicklerflasche befindlichen Wasserstoffperoxid-Zubereitung vermischt. Auf diese Weise wird die Anwendungsmischung in der Entwicklerflasche hergestellt. Das Aufbringen auf dem Haar erfolgt dann über eine kleine Tülle oder Auslassöffnung am Kopf der Entwicklerflasche. Die Tülle bzw. Ausfassöffnung wird nach dem Verschütteln geöffnet, und die Anwendungsmischung kann durch Drücken der flexiblen Entwicklerflasche entnommen werden.

Die Verwendung der Entwicklerflasche erfordert vom Anwender eine gewisse Routine, so dass mancher Anwender die Herstellung der Anwendungsmischung in einer Anmisch-Schale und die Applizierung mittels eines Pinsels bevorzugt.

Bei Herstellung der Anwendungsmischung in einer Schale werden beide Komponenten - die erste Zubereitung, welche das Oxidationsmittel enthält und die zweite Zubereitung mit Alkalisierungsmittel und/oder Oxidationsfarbstoffvorprodukten - komplett in eine Schale oder ein ähnliches Gefäß überführt und dort beispielsweise unter Zuhilfenahme eines Pinsels verrührt. Die Entnahme der Anwendungsmischung erfolgt dann über den Pinsel aus der Anmisch-Schale. Bei dieser Form der Anwendung ist der Einsatz einer voluminösen und teuren Entwicklerflasche nicht notwendig, und es wird nach wie vor nach preiswerten und materialsparenden Verpackungsformen für die Oxidationsmittelzubereitung gesucht.

Als preiswerte Verpackungsform mit geringem Materialverbrauch bietet sich in diesem Zusammenhang Verpackungen in Taschen- oder Beutelform an, welche in der Regel aus Kunststoff-Folien oder auch Metall-Folien gefertigt werden.

Eine derartige Verpackung kann beispielsweise durch Verklebung oder Heißverpressung von zwei übereinander liegenden Kunststoff-Folien hergestellt werden, wobei die Verklebung an allen Rändern der Folien erfolgt. Der durch das Verkleben hergestellte Innenraum der Verpackung (d.h. des Kunststoffbeutels) kann dann mit der gewünschten kosmetischen Zubereitung befüllt werden. Die Öffnung der Verpackung kann durch Aufreißen oder Aufschneiden des Kunststoffbeutels erfolgen.

Die Abfüllung von Oxidationsmittelzubereitungen in derartige Verpackungen ist jedoch mit Problemen verbunden, deren Ursache in der Reaktivität des Oxidationsmittels begründet liegt. Bei Oxidationsmitteln handelt es sich um eine hochreaktive Substanzen, welche sich - abhängig von den Lagerbedingungen sowie ggf. von der Anwesenheit zersetzend wirkender Verunreinigungen - in kleinen Anteilen unter Ausbildung von Sauerstoff (d.h. von Gas) zersetzen.

Die aus dem Stand der Technik bekannten Entwicklerflaschen werden in der Regel nur maximal zur Hälfte, üblicherweise lediglich zu einem Drittel ihres Innenvolumens mit der Oxidationsmittelzusammensetzung befüllt. In der Regel werden Entwicklerflaschen aus Polyethylen hergestellt. Da Polyethylen sowohl gegenüber Wasserdampf als auch gegenüber anderen Gasen durchlässig ist, entsteht in der Entwicklerflasche kein oder nur sehr geringer Überdruck. Darüberhinaus sind Entwicklerflaschen üblicherweise mit stabilen, dicken Wänden und einem stabilen Schraub-Verschluss versehen, so dass die Diffusion des Wasserdampfes bzw. der Gase durch die Dicke der Wände reduziert wird und eine in geringem Ausmaß stattfindende Druckerhöhung innerhalb der Flasche keine negativen Auswirkungen hat.

Im Gegensatz hierzu werden beutelförmige Verpackungen jedoch üblicherweise komplett mit der flüssigen Zubereitung befüllt, und im befüllten Beutel existiert praktisch kein überstehender Luftraum. Zudem sollen derartige Verpackungen flexibel sein, und beim Öffnen (z.B. Aufreißen oder Aufschneiden) soll kein unkontrollierter Austritt der Zubereitung erfolgen. Aus diesem Grund sollte bei der Verpackung von flüssigen Zubereitungen die Entstehtung von Überdruck in der Verpackung nach Möglichkeit vermieden werden.

Befindet sich nun eine Oxidationsmittelzusammensetzung in einer derartigen Verapckung, so kann das während der Lagerung entstehende Gas (Sauerstoff) zu einem Aufblähen der Verpackung führen. Da die Ränder der Verpackung überlicherweise nur verklebt sind, führt ein starkes Aufblähen schlimmstenfalls zu einem Platzen der Verpackung. Aus diesen Gründen ist bei der Lagerung von Oxidationsmittel-haltigen Zusammensetzungen die Wahl des Folienmaterials, aus der die Verpackung besteht, von großer Bedeutung.

Verpackungen, die aus reinem Kunststoff wie beispielsweise Polyethylen oder Polypropylen bestehen, sind sowohl gegenüber Wasserdampf als auch gegenüber Gasen durchlässig. Bei Lagerung einer Oxidationsmittel-haltigen Zubereitungen in einer Verpackung aus Polyethylen oder Polypropylen erfolgt deshalb kein Aufblähen der Verpackung. Aufgrund der hohen Durchlässigkeit der vergleichsweise dünnen Folie der Verpackung gegenüber Wasserdampf verringert sich jedoch der Wassergehalt der Zubereitung. Wird die Zubereitung in der Verpackung für einige Wochen bis Monate gelagert, übersteigt der Wasserverlust den für eine ausreichende Lagerstabilität erlaubten Höchstwert.

Die Herstellung geeigneter Verpackungen für Wasserstoffperoxid-haltige Formulierungen ist eine Herausforderung. Die oben dargestellten Eigenschaften für die Durchlässigkeit von Sauerstoff und Wasserdampf müssen so eingestellt sein, dass eine ausreichende Lagerstabilität gegeben ist. Die Schichtdicke der Folie sollte aus Gründen des Umweltschutzes und aus Gründen der Resoucenschonung möglichst gering gehalten werden. Darüber hinaus hat die Schichtdicke selbstverständlich auch einen Einfluss auf die Herstellungskosten. Vor diesem Hintergrund sind dünne Schichten gewünscht, die jedoch nicht immer eine ausreichende mechnische Festigkeit gewährleisten. Wenn verschiedene Materialien in einer Mehrschichtfolie kombiniert werden, um einem breiten Anforderungsprofil genüge zu tun, muss auch die Herstellbarkeit der Mehrschichtfolie gewährleistet sein. Bestimmte Materialien lassen sich nämlich nicht miteinander kombinieren, da die Kohäsion zwischen Schichten nicht immer ausreichend ist oder da deren Verarbeitungstemperaturen so unterschiedlich sein können, dass eine gemeinsame Verarbeitung schwierig ist.

Schießlich sind die Folienmaterialien gerade bei der Aufbewahrung eines Mehrkomponentensystems von großer Bedeutung, da Stoffe aus dem Mehrkomponentensystem in die Folien hineindiffundieren können und eine Ablösung von Schichten, die die Folie bilden, befördern können. Die Wahl der Komponenten eines Wasserstoffperoxid-haltigen Formulierung hat somit auch einen Einfluss auf die Wahl der Verpackung.

Die Aufgabe der vorliegenden Anmeldung bestand darin, Wasserstoffperoxid-haltige Formulierungen so zu verpacken, dass die mechanische Festigkeit der Verpackung ausreichend groß ist, um eine sichere Lagerung zu ermöglichen, dass aber eine leichte Zugänglichkeit zu den Inhaltsstoffen gewährleistet ist.

DE 102015223838 A1 betrifft Produkte zur oxidativen Färbveränderung, die eine Verpackung in Form eines Sachets und eine darin verpackte Oxidationsmittelzubereitung umfassen. Im Beispielteil der DE 102015223838 A1 wird zunächst die Herstellung einer Verpackung aus Folienmaterial beschrieben, die anschließend mit einer kosmetischen Zubereitung befüllt wird.

Überraschenderweise hat sich nun herausgestellt, dass Oxidationsmittelhaltige Zusammensetzungen verpackt werden können, bei denen die Wasserdampfdurchlässigkeit gering ist und ein Aufblähen dadurch verringert werden kann, indem die Folie ein bestimmtes Maß an Sauerstoffdurchlässigkeit ermöglicht. Die Folien bestehen aus einem speziellen Folien-Verbundsystemen und besitzen zusätzlich eine Barriere-Schicht. Dadurch, dass die Wasserdampfdurchlässigkeit verringert wird, jedoch die Sauerstoffdurchlässigkeit auf ein ausreichend hohes Mass eingestellt wird, wird die Neigung des Aufblähens durch Sauerstoff, der sich aus dem Wasserstoffperoxid bildet, verringert und die mechanische Festigkeit über die Zeit erhöht.

Gegenstand der vorliegenden Erfindung ist ein kosmetisches Produkt zur Veränderung der natürlichen Farbe keratinischer Fasern, insbesondere menschlicher Haare, umfassend
(i) mindestens eine Verpackung (VP), umfassend mindestens eine mehrlagige Folie (F), welche mindestens eine erste Polymerschicht (P1), mindestens eine zweite Polymerschicht (P2) sowie mindestens eine Barriereschicht (BS) enthält, und
(ii) mindestens eine kosmetische Zusammensetzung (KM), welche in der Verpackung (VP) verpackt ist und enthält:
   a) mindestens eine oxidierende Verbindung,
   b) mindestens einen C₈-C₃₀-Alkohol, und
   c) mindestens ein nichtionisches Tensid,
wobei die erste Polymerschicht (P1) aus Polyethylenterephthalat, gebildet ist; die zweite Polymerschicht (P2) aus Polyethylen gebildet ist; und die Barriereschicht (BS) aus einem mit einer SiOₓ-Schicht versehenen Polyethylenterephthalat, gebildet ist.

Unter keratinischen Fasern, keratinhaltigen Fasern oder Keratinfasern sind Pelze, Wolle, Federn und insbesondere menschliche Haare zu verstehen. Obwohl die erfindungsgemäßen Mittel in erster Linie zum Aufhellen und Färben von Keratinfasern geeignet sind, steht prinzipiell einer Verwendung auch auf anderen Gebieten nichts entgegen.

Bei dem erfindungsgemäßen Produkt handelt es sich um ein Produkt für die oxidative Farbveränderung von keratinischen Fasern, d.h. um ein Produkt, welches auf dem menschlichen Kopf angewendet wird, um eine oxidative Färbung, eine Aufhellung, eine Blondierung, eine Bleiche oder eine Nuancierung der Haare zu erzielen. Unter Nuancierung wird in diesem Zusammenhang eine Färbung verstanden, bei welcher das Farbresultat heller als die Ausgangshaarfarbe ist. Dass das Produkt "zur Veränderung der natürlichen Farbe" verwendet werden soll, soll bedeuten, dass das Produkt entweder nur ein Oxidationsmittel zur Bondierung umfasst, oder dass das Produkt ein Oxidationsmittel umfasst, dass mit einem nicht zur Erfindung gehörigen Kuppler verwendet wird, um eine Farbveränderung zu erzielen, oder dass das Produkt mit einem nicht zur Erfindung gehörenden Farbstoff zur weiteren Tönung verwendet wird.

Weiterhin wird unter dem Begriff "Verpackung" erfindungsgemäß eine Verpackung verstanden, welche bevorzugt in Form eines Sachets vorliegt. Ein Sachet ist eine kleine Verpackung in Taschen- oder Beutelform, welche oft bei der Verpackung von Kosmetika eingesetzt wird. Das Fassungsvermögen der Verpackung, insbesondere des Sachets, kann beispielsweise 5 bis 1000 ml, bevorzugt 10 bis 200 ml und besonders bevorzugt 20 bis 50 ml betragen.

Zudem wird unter einer mehrlagigen Folie (F) im Rahmen der vorliegenden Erfindung eine dünne, flächige und aufwickelbare Bahn aus der mindestens einen Polymerschicht (P1) und der mindestens einen Polymerschicht (P2) verstanden. Diese mehrlagige Folie (F) bildet die Wandung der Verpackung (VP) aus. Die Verpackung enthält zusätzlich eine Barriereschicht (BS), welche den Durchtritt von Wasserdampf und anderen Gasen, wie beispielsweise Sauerstoff, gezielt ermöglicht oder vermindert.

Das erfindunsgemäße kosmetische Produkt umfasst als ersten Bestandteil eine Verpackung (VP), welche mindestens eine mehrlagige Folie (F) umfasst. Diese Folie enthält mindestens eine erste Polymerschicht (P1), mindestens eine zweite Polymerschicht (P2) sowie mindestens eine Barriereschicht (BS). Diese mehrschichtige Folie stellt die Wandung bzw. die Außenhülle der Verpackung dar. Wie zuvor beschrieben wird eine derartige Verpackung üblicherweise durch Verkleben, Verpressen oder Verschweißen von zwei übereinanderliegenden Folienstücken hergestellt (wobei die Verpackung (VP) gleichzeitig mit der kosmetischen Zusammensetzung (KM) befüllt wird) , d.h. eine derartige Verpackung ist an allen Rändern verschlossen. Geöffnet werden kann diese Verpackung beispielsweise durch Aufreißen oder Aufschneiden.

Die Dicke der mehrlagigen Folie (F) bestimmt die mechanischen Eigenschaften und die Festigkeit der Folien. Sie soll hierbei so gestaltet sein, dass eine ausreichende mechanische Stabilität vorliegt, gleichzeitig aber auch die Folie (F) - und damit die aus der Folie hergestellte Verpackung (VP) - so flexibel ist, dass eine vollständige Entnahme der kosmetischen Zusammensetzung (KM) aus der geöffneten Verpackung (VP) durch Zusammenpressen bzw. Auspressen ermöglicht wird. Diesen Anforderungen wird eine Folie dann gerecht, wenn die Folie (F) eine bestimmte Gesamtdicke aufweist. Bevorzugte Ausführungsformen der vorliegenden Erfindung sind daher dadurch gekennzeichnet, dass die mindestens eine mehrlagige Folie eine Gesamtdicke von 28 µm bis 220 µm, bevorzugt von 52 µm bis 180 µm, bevorzugter von 80 µm bis 140 µm, aufweist. Unter der Gesamtdicke der Folie (F) wird im Sinne der vorliegenden Erfindung die Summe der Dicken aller Einzelschichten verstanden, aus welchen die Folie (F) besteht.

Die Anordnung der Schichten (P1), (P2) sowie (BS) innerhalb der mehrlagige Folie (F) kann unterschiedlich sein. Weiterhin ist es auch möglich, dass die Folie (F) neben den zuvor angeführten Schichten noch weitere Schichten umfasst. Zudem ist es erfindungsgemäß vorteilhaft, wenn alle zuvor angeführten Schichten jeweils parallel zu den Oberflächen der Folie (F) orientiert sind, also alle Schichten die gleiche Orientierung aufweisen. Enthält die mehrlagige Folie (F) die zuvor beschriebenen drei Schichten (P1), (P2) und (BS), wären folgende Anordnungen der Schichten möglich (von Innenraum (steht in Konakt mit der kosmetischen Zusammensetzung (KM)) zur Außenseite betrachtet):
a) *Innenraum* - Schicht (P1) - Schicht (P2) -Barriereschicht (BS) - *Außenseite*,
b) *Innenraum* - Schicht (P1) - Barriereschicht (BS) - Schicht (P2) - *Außenseite*,
c) *Innenraum* - Schicht (P2) - Schicht (P1) -Barriereschicht (BS) - *Außenseite*,
d) *Innenraum* - Schicht (P2) -Barriereschicht (BS) - Schicht (P1) - *Außenseite*,
e) *Innenraum* - Barriereschicht (BS) - Schicht (P1) -Schicht (P2) - *Außenseite*,
f) *Innenraum* - Barriereschicht (BS) - Schicht (P2) - Schicht (P1) - *Außenseite*.

Erfindungsgemäß bevorzugt ist es jedoch, wenn die Barriereschicht (BS) zwischen der ersten Polymerschicht (P1) und der zweiten Polymerschicht (P2) angeordnet ist, wobei sich die zweite Polymerschicht (P2) an der Außenseite der Verpackung befindet. In diesem Fall besteht die mehrschichtige Folie (F) aus drei Schichten, wobei die Schicht (P1) zuinnerst liegt und Kontakt mit der kosmetischen Zusammensetzung (KM) hat. Die Schicht (P1) hat Kontakt mit der Barriereschicht (BS), und die Barriereschicht (BS) hat wiederum Kontakt mit der Schicht (P2). Bei dieser Schicht grenzen die Schichten (P1) und (P2) nicht aneinander, sondern werden durch die Barriereschicht (BS) getrennt. Der besondere Vorteil dieser Anordnung liegt darin, dass die - oft sehr dünne - Barriereschicht (BS) sich weder an der inneren noch an der äußeren Oberfläche der mehrlagigen Folie (F) befindet, sondern in Richtung Innenseite durch die polymere Schicht (P1) und in Richtung Außenseite durch die polymere Schicht (P2) geschützt ist. Auf diese Weise kann bei dieser Anordnung ein mechanischer Abrieb oder eine mechanische Zerstörung der Barriereschicht (BS) bestmöglich vermieden werden. Es ist daher im Rahmen der vorliegenden Erfindung vorteilhaft, wenn die mindestens eine mehrlagige Folie (F) die mindestens eine Barriereschicht (BS) zwischen der mindestens einen ersten Polymerschicht (P1) und der mindestens einen zweiten Polymerschicht (P2) enthält. Der Einsatz derartiger Verpackungen, hat sich als besonders vorteihaft in Bezug auf die erhöhte Lagerstabiliät erwiesen, da diese Anordnung weder eine Aufblähung noch eine Delaminierung bei längerer Kontaktzeit mit einer Oxidationsmittel-haltigen Zusammensetzung zeigt. In einer weiteren bevorzugten Ausführungsform ist die Barriereschicht (BS) ebenfalls zwischen den beiden Polymerschichten P1 und P2 angeordnet, wobei sich jedoch die erste Polymerschicht (P1) an der Außenseite der Verpackung befindet.

Unter Außenseite der Verpackung (VP) ist erfindungsgemäß diejenige Seite der Verpackung zu verstehen, welche nicht mit der kosmetischen Zusammensetzung (KM), sondern mit der Umgebung in Kontakt kommt. Der Einsatz derartiger Verpackungen, hat sich als besonders vorteihaft in Bezug auf die erhöhte Lagerstabiliät erwiesen, da diese Anordnung weder eine Aufblähung noch eine Delaminierung bei längerer Kontaktzeit mit einer Oxidationsmittel-haltigen Zusammensetzung zeigt.

Gemäß der vorliegenden Erfindung ist die mindestens eine erste Polymerschicht (P1) aus Polyethylenterephthalat gebildet. Unter dem Begriff "gebildet ist" wird erfindungsgemäß verstanden, dass die Polymerschicht mindestens 70 Gew.-%, vorzugsweise mindestens 80 Gew.-%, bevorzugt mindestens 90 Gew.-%, insbesondere mindestens 99 Gew.-%, jewiels bezogen auf das Gesamtgewicht der Polymerschicht (P1), der zuvor angeführten Verbindungen enthält.

Polyethylenterephthalat (PET) ist ein Polymer aus der Gruppe der Polyester. Die Herstellung von Polyethylenterephthalat kann beispielsweise durch Umesterung von Dimethylterephthalat mit Ethylenglycol bei höheren Temperaturen erfolgen. Bei dieser Umesterungsreaktion wird Methanol abgespalten, welches durch Destillation entfernt wird. Das entstehende Bis(2-hydroxyethyl)-terephthalat wird durch Polykondensation zu PET umgesetzt, wobei wieder Ethylenglycol entsteht. Eine weitere Herstellmethode von Polyethylenterephthalat ist die direkte Polykondensation von Ethylenglykol und Terephthalsäure bei hohen Temperaturen unter Abdestillation des entstehenden Wassers. Polyethylenterephthalat zeichnet sich durch eine besonders hohe mechnische Festigkeit aus. Wenn die PET-Schicht die Außenschicht bildet, bietet dies ferner den Vorteil, dass die Schicht darunter bedruckt werden kann, ohne dass der Druck abgerieben werden kann. Die PET-Schicht ist transparent und bietet für die Druckschicht eine mechanische Schutzschicht.

Gemäß einer bevorzugten Ausführungsform der vorliegenden Erfindung beträgt die Schichtdicke der ersten Polymerschicht (P1) 4 µm bis 50 µm, bevorzugt 5 µm bis 35 µm, bevorzugter 6 µm bis 20 µm. Die gemäß der bevorzugten Ausführungsform verwendete Schichtdicke der PET-Schicht ist mit besonderen Vorteilen verbunden, was mit allgemeinen Eigenschaften von PET zusammenhängt. PET zeichnet sich durch eine hohe Formfestigkeit/Steifheit aus. Wenn als erste Polymerschicht (P1) PET mit diesen Schichtdicken gewählt wird, bietet dies für die Folie eine vorteilhafte mechnische Formfestigkeit. Gleichzeitig kann die Gesamtdicke der Folie gering gehalten werden, dass eine material- und resourcenschonende Folie bereitgestellt werden kann.

Weiterhin umfasst die mehrlagige Folie (F), aus der die Verpackung hergestellt wird, eine zweite Polymerschicht (P2) aus einem zweiten polymeren Material. Die mindestens eine zweite Polymerschicht (P2) ist aus Polyethylen gebildet. Unter dem Begriff "ist gebildet" wird erfindungsgemäß verstanden, dass die Polymerschicht mindestens 70 Gew.-%, vorzugsweise mindestens 80 Gew.-%, bevorzugt mindestens 90 Gew.-%, insbesondere mindestens 99 Gew.-%, jewiels bezogen auf das Gesamtgewicht der Polymerschicht (P2), der zuvor angeführten Verbindungen enthält.

Bei dem zweiten polymeren Material der zweiten Schicht (P2) der mehrlagigen Folie (F) handelt es sich um Polyethylen. Polyolefine sind Polymere, die aus Alkenen wie Ethylen, Propylen, 1-Buten oder Isobuten durch Kettenpolymerisation hergestellt werden. Bei den Polyolefinen handelt es sich um gesättigte Kohlenwasserstoffe. Es sind teilkristalline Thermoplaste, die sich leicht verarbeiten lassen. Sie zeichnen sich durch gute chemische Beständigkeit aus. Bei Folienanwendungen sind Polyethylen und Polypropylen sehr weit verbreitet. Erfindungsgemäß wird für die zweite Schicht (P2) daher Polyethylen verwendet. Polyethylen wird durch Polymerisation von Ethylen unter Einsatz verschiedener Katalysatoren hergestellt. So kann Polyethylen beispielsweise durch Polymerisation von Ethylen in der Gasphase oder in Suspension hergestellt werden. Die Regelung der mittleren relativen Molmasse kann beispielsweise durch Einstellung eines bestimmten Wasserstoff-Partialdruckes während der Polymerisation des Ethylens erfolgen. Die Verarbeitung von Polyethylen kann beispielsweise durch Extrusions- und Streckblasformen, oder durch Pressen, Kalandrieren, Thermoformen und Kaltumformen erfolgen.

Die zweite Polymerschicht (P2) dient als Stützschicht. Polyethylen hat zwar den Nachteil, für Sauerstoff und Wasserdampf durchlässig zu sein, hat aber den Vorteil, kostengünstig zu sein und aufgrund des niedrigen Schmelzpunktes - niedriger als der von Polypropylen - leicht und energiesparend verarbeitbar zu sein.

Gemäß einer bevorzugten Ausführungsform der vorliegenden Erfindung weist die zweite Polymerschicht (P2) eine bestimmte Schichtdicke auf. Gemäß der bevorzugten Ausführungsform weist die zweite Polymerschicht (P2) eine Schichtdicke von von 20 µm bis 150 µm, bevorzugt von 30 µm bis 110 µm, bevorzugter von 40 µm bis 90 µm, auf. Besonders bevorzugt weist die zweite Polymerschicht (P2) eine höhere Schichtdicke auf als die erste Polymerschicht (P1).

Die Polymerschichten (P1) und (P2) der mehrlagigen Folie (F) umfassen organische polymere Materialien, welche in der Regel nur eine ungenügende Sperrwirkung gegenüber Gasen und Wasserdampf besitzen. Wird die Oxidationsmittel-haltige Zusammensetzung (KM) in einer Verpackung (VP) aus einer mehrschichtige Folie (F) verpackt, welche lediglich die beiden organischen Polymer-Schichten (P1) und (P2) umfasst, kann Wasserdampf ungehindert austreten, so dass sich der Wassergehalt in der Zusammensetzung (KM) bei längerer Lagerung in nicht akzeptabler Weise verändert. Um den unkontrollierten Austritt von Wasserdampf aus der Verpackung (VP) gezielt zu minimieren, werden die organischen Polymerschichten (P1) und (P2) daher im Verbund mit einer Barriereschicht (BS) eingesetzt.

Die Barriereschicht (BS) besitzt eine Durchtrittsperrwirkung für Gase und Wasserdampf. Damit ist erfindungsgemäß gemeint, dass die Barriereschicht (BS) die Permeationsrate von Wasserdampf und von Gasen durch die Folie hindurch verringert und steuert. Eine erfindungsgemäße Folie (F), welche neben den Schichten (P1) und (P2) eine Barriereschicht (BS) besitzt, weist also gegenüber einer vergleichbaren Folie (mit gleicher Gesamtdicke), welche lediglich die beiden Schichten (P1) und (P2), jedoch keine Barriereschicht (BS) besitzt, eine verminderte Wasserdampfdurchlässigkeit und eine verminderte Gasdurchlässigkeit auf.

Bei der Barriereschicht (BS) handelt es sich beispielsweise um eine dünne Schicht, welche ein anorganisches Material umfasst, wobei das anorganische Material mit Hilfe von Vakuumbeschichtungstechniken (z.B. PVD "physical vapour deposition" oder CVD "chemical vapour deposition") auf organische Polymerschichten aufgetragen werden kann.

Erfindungsgemäß ist die Barriereschicht (BS) aus einem mit einer SiOₓ-Schicht versehenen Polyethylenterephthalat gebildet. Die SiOₓ-Sicht ist besonders gut kompatibel mit der angrenzenden Polyethylenschicht.

Gemäß einer besonders bevorzugten Ausführungsform der vorliegenden Erfindung ist die Polyethylenterephthalatschicht der Barriereschicht (BS) Teil der ersten Schicht (P1). Mit anderen Worten wird das SiOₓ auf die erste Schicht (P1) aufgedampft. Die Schichtdicke der PET-Schicht entspricht dann der Schichtdicke der PET-Schicht, die Teil der Barriereschicht (BS) ist, und der Schichtdicke, die Teil der ersten Schicht (P1) ist.

Der Polyethylenterephthalatfilm ist mit einer SiOₓ-Schicht versehen. Das Siliciumoxid wird auf den Polyethylenterephthalatfilm aufgedampft und ist in erster Linie ursächlich für die Sperrwirkung. Das Verhältnis der Schicktdicke von SiOₓ zu Polyethylenterephthalat liegt gemäß einer bevorzugten Ausführungsform der vorliegenden Erfindung bei 1 : 1000 bis 1 : 10, bevorzugt von 1 : 500 bis 1 : 50, bevorzugter von 1 : 200 bis 1 : 100.

Die Herstellung von Folien mit Barriereschichten umfassend anorganisches Material ist bekannt. Auch die erfindungsgemäß verwendete mehrlagige Folie (F) kann durch ein Verfahren hergestellt werden, das für die Herstellung bekannter Folien mit Barriereschichten im Stand der Technik angewendet wird, wie beispielsweise in den Schriften EP 1036813 A1, EP 2371539 A1 und EP 1541340 A1 beschrieben.

Die Barriereschicht (BS) kann zusätzlich auch eine dünne Schicht aus anorganisch-organischen Hybridpolymeren umfassen. Diese Polymere sind in der Literatur unter dem Fachbegriff ORMOCER-Polymere bekannt. Ein typisches ORMOCER-Polymer lässt sich beipsielsweise unter hydrolytischer Polykondensation eines organofunktionellen Silans mit einer Aluminiumverbindung und gegebenenfalls mit einer anorganischen Oxidkomponente herstellen. Entsprechende Synthesen sind beispielsweise in der Schrift EP 0792846 B1 offenbart, auf die an dieser Stelle vollinhaltlich Bezug genommen wird. Anorganisch-organische Hybridpolymere (ORMOCER-Polymere) weisen sowohl anorganische als auch organische Netzwerkstrukturen auf. Der Aufbau der anorganischen silikatischen Netzwerkstruktur kann im Sol-Gel-Prozess über die gesteuerte Hydrolyse und Kondensation von Alkoxysilanen erfolgen. Indem zusätzlich Metallalkoxide in den Sol-Gel-Prozess einbezogen werden, kässt sich das silikatische Netzwerk gezielt modifizieren. Durch Polymerisation von organofunktionellen Gruppen, welche durch die Organoalkoxylane in das Material eingebracht werden, wird zusätzlich ein organisches Netzwerk aufgebaut. Die auf diese Weise hergestellten ORMOCER-Polymere können beispeilsweise mittels herkömmlicher Applikationstechniken (Sprühen, Streichen usw) auf die Schichten (P1) und/oder (P2) aufgetragen werden.

Je dicker die Barriereschicht (BS) ist, desto größer bzw. stärker ist die Durchtrittsperrwirkung für Gase und Wasserdampf. Die Dicke der Barriereschicht (BS) kann daher in Abhängigkeit von der gewünschten Sperrschichtwirkung gewählt werden. Gemäß einer bevorzugten Ausführungsform der vorliegenden Erfindung weist die mindestens eine Barriereschicht (BS) eine Schichtdicke von 4 µm bis 20 µm, bevorzugt von 5 µm bis 18 µm, bevorzugter von 6 µm bis 15 µm, auf.

Das Material, der Aufbau und die Schichtdicken bestimmen die Durchlässigkeitswerte der Folie. Die mehrlagige Folie (F) der Verpackung des erfindungsgemäßen kosmetischen Produkts zeichnet sich durch vorteilhafte Eigenschaften bezüglich der Sauerstoffdurchlässigkeit und der Wasserdampfdurchlässigkeit aus. Die mehrlagige Folie zeigt eine Sauerstoffdurchlässigkeitsrate (OTR) bei 23 °C und 50 % relativer Luftfeuchtigkeit von 0,1 bis 5 cc/m²/d/bar, bevorzugt von 0,2 bis 3,5 cc/m²/d/bar, bevorzugter von 0,5 bis 2,5 cc/m²/d/bar, und eine Wasserdampfdurchlässigkeit bei 38 °C und 100 % relativer Luftfeuchtigkeit von 0,1 bis 5 g/m²d, bevorzugt von 0,2 bis 3,5 g/m²d, bevorzugter von 0,5 bis 2,5 g/m²d.

Erfindungsgemäß werden die Durchlässigkeitswerte der Folie (F) vorteilhaft eingestellt. Die Folie (F) verleiht so der Verpackung vorteilhafte Barriereeigenschaften, insbesondere im Hinblick auf die Durchlässigkeit für Wasserdampf (*engl.: Water Vapor Transmission Rate; WVTR*; gemessen in der Einheit g/(m²d) bzw. g/(m²24h)) gemessen nach der Methode ASTM F 1249 bei 38 °C Umgebungstemperatur und 100 % relativer Luftfeutigkeit, und für Sauerstoff (*engl.: Oxygen Transmission Rate; OTR,* gemessen in cm³/(m²d bar) bzw. cm³/(m² 24h) - wobei cm³ gleichbedeutend mit cc ist - bei einem Atmospharendruck von 1 bar) gemessen nach der Methode ASTM D 3985 bei 23 °C Umgebungstemperatur und 50 % relativer Luftfeutigkeit.

Neben den bislang beschriebenen Schichten (P1), (P2) und (BS) kann die mehrlagige Folie (F) zusätzlich auch noch eine oder mehrere weitere Schichten umfassen. Bei diesen weiteren Schichten kann es sich beispielsweise um Zwischenschichten und/oder Klebeschichten handeln. Es ist daher erfindungsgemäß bevorzugt, wenn die mindestens eine mehrlagige Folie (F) zusätzlich mindestens eine weitere Schicht, ausgewählt aus der Gruppe von Zwischenschichten (SZ), Klebeschichten (SK) und deren Mischungen, enthält.

Beispielsweise können die Folien (F) weitere Zwischenschichten (SZ) besitzen, um die mechanische Stabilität zu erhöhen. Zwischenschichten können auch die Permeation von Polymeren oder verbliebenen Monomeren aus einer Polymerschicht in die kosmetische Zusammensetzung (KM) verhindern oder minimieren.

Um die Verbundfestigkeit zu erhöhen, können die Folien zusätzlich auch noch eine oder mehrere Klebeschichten (SK) umfassen, um eine Delaminierung (d.h. ein Ablösen oder die Bildung eines Luftraums) zwischen zwei Schichten zu vermindern bzw. zu verhindern.

Ein besonders bevorzugtes erfindungsgemäßes Produkt ist dadurch gekennzeichnet, dass die mehrlagige Folie (F) neben der ersten Polymerschicht (P1), der zweiten Polymerschicht (P2) und der Barriereschicht (BS) zusätzlich noch eine oder mehrere weitere Schichten enthält, welche aus Zwischenschichten (SZ) und/oder Klebeschichten (SK) ausgewählt sind.

Enthält die mehrlagige Folie (F) zusätzlich zu den Schichten (P1), (P2) und (BS) auch noch weitere Schichten, sind folgende Anordnungen der Schichten möglich (von Innenraum (steht in Konakt mit der kosmetischen Zusammensetzung (KM)) zur Außenseite betrachtet):
a) *Innenraum* - Schicht (P1) - erste Klebeschicht (SK1) - Schicht (P2) - zweite Klebeschicht (SK2) - Barriereschicht (BS) - *Außenseite*,
b) *Innenraum* - Schicht (P1) - Klebeschicht (SK1) - Schicht (P2) - Barriereschicht (BS) - *Außenseite*,
c) *Innenraum* - Schicht (P1) - Schicht (P2) - zweite Klebeschicht (SK2) - Barriereschicht (BS) - *Außenseite*,
d) *Innenraum* - Barriereschicht (BS) - erste Klebeschicht (SK1) - Schicht (P1) - zweite Klebeschicht (SK2) - Schicht (P2) - *Außenseite*,
e) *Innenraum* - Barriereschicht (BS) - Klebeschicht (SK) - Schicht (P1) - Schicht (P2) - *Außenseite*,
f) *Innenraum* - Barriereschicht (BS) - Schicht (S1) - Klebeschicht (SK) - Schicht (P2) - *Außenseite*,
g) *Innenraum* - Schicht (P1) - erste Klebeschicht (SK1) - Barriereschicht (BS) - zweite Klebeschicht (SK2) - Schicht (P2) - *Außenseite*,
h) *Innenraum* - Schicht (P1) - Klebeschicht (SK) - Barriereschicht (BS) - Schicht (P2) - *Außenseite*,
i) *Innenraum* - Schicht (P1) - Barriere Schicht (BS) - Klebeschicht (SK) - Schicht (P2) - *Außenseite*

In jedem Fall soll die Folie so gestaltet sind, dass zwischen den Filmen eine genügende Haftfestigkeit gegeben ist. Gemäß einer bevorzugten Ausführungsform der vorliegenden Erfindung beträgt die Haftfestigkeit (*engl.: bond strength*) der Folie 0,1 bis 10 N/15mm, bevorzugt 1 bis 8 N/15mm, bevorzugter 1,5 bis 5 N/15mm. Gemessen wird dies durch die Methode ASTM F-904. Bei der Haftfestigkeit handelt es sich um ein physikalisches Maß der Adhäsionskraft zwischen den Schichten. Bezogen ist die Haftfestigkeit auf die zwei Schichten einer Folie mit der geringsten Haftfestigkeit zwischen zwei Schichten ein und derselben Folie. Die gemäß der Erfindung eingestellten Haftfestigkeiten führen zu einer vorteilhaften mechanischen Stabilität über die Lagerzeit des verpackten kosmetischen Produkts.

Auch die Festigkeit zwischen zwei verbundenen (gesiegelten oder versiegelten) Folien soll ein ausreichendes Maß einhalten. Gemäß einer bevorzugten Ausführungsform der vorliegenden Erfindung beträgt die Siegelnahtfestigkeit (*engl.: seal strength*) der Verpackung (VP) 10 bis 40 N/15mm, bevorzugt 15 bis 35 N/15mm, bevorzugter 20 bis 30 N/15mm, unter den Bedingungen 150 °C, 2,54 cm (1") und 4 kg/cm². Gemessen wird die Siegelnahtfestigkeit nach der Methode ASTM F-88 unter den genannten Bedingungen. Die Herausforderung bei Verpackungen ist stets die Sicherstellung der mechanischen Haltbarkeit der Verpackung bei gleichzeitig leichter Zugänglichkeit des Inhalts durch den Verwender. Die Einstellung der Siegelnahtfestigkeit auf diese Werte ermöglicht die Erreichung dieser beiden Ziele.

Unter einer Siegelnaht wird eine Naht verstanden, durch die die Verpackung verschlossen wird. Üblicherweise liegen für das Verschließen der Verpackung zwei Folien übereinander, die durch eine Kraft senkrecht zur Folienoberfläche zusammengedrückt werden. Durch ein Erhitzen der Folien in dem Bereich, der zusammengedrückt wird, können Teile der zusammengedrückten Bereiche miteinander verschmelzen, so dass die Folien verschweißt vorliegend. Zwischen den zusammengedrückten Folien kann sich auch ein Klebstoff befinden, der die Naht festigt.

Das erfindungsgemäße Produkt umfasst als zweiten Bestandteil eine kosmetische Zusammensetzung (KM), die in der Verpackung (VP) verpackt ist, und die mindestens ein Oxidationsmittel, einen linearen C₈- bis C₃₀-Alkohol sowie ein nichtionisches Tensid umfasst.

Verwendungszweck des erfindungsgemäßen Produktes ist die oxidative Farbveränderung. Hierfür wird - wie bereits zuvor beschrieben - üblicherweise eine kosmetische Zusammensetzung (KM), welche ein Oxidationsmittel enthält, mit einer zweiten, getrennt von (KM) konfektionierten Zubereitung (B) vermischt. Auf diese Weise wird das anwendungsbereite oxidative Farbänderungsmittel hergestellt. Abhängig davon, ob mit der oxidativen Farbänderung eine Blondierung, Aufhellung oder Färbung erzielt werden soll, kann die Zubereitung (B) verschiedene Inhaltsstoffe enthalten. Wenn eine reine Aufhellung bzw. Blondierung erzielt werden soll, enthält die Zubereitung (B) mindestens ein Alkalisierungsmittel. Wird eine oxidative Färbung gewünscht, so sind in der Zubereitung (B) neben dem Alkalisierungsmittel oft auch Oxidationsfarbstoffvorpodukte enthalten. Um eine ausreichend schnelle Vermischbarkeit der Zubereitungen (KM) und (B) zu gewährleisten, handelt es üblicherweise sowohl bei der Zubereitung (KM) als auch bei der Zubereitung (B) um fließfähige, wässrige oder wasserhaltige Zubereitungen.

Bei der Zubereitung (KM) handelt es sich erfindungsgemäß um eine wässrige Zubereitung. Der Wassergehalt der Zubereitung (KM) kann - bezogen auf das Gesamtgewicht der Zubereitung (KM) - beispielsweise bei 60 bis 97 Gew.-%, vorzugsweie bei 75 bis 93 Gew.-%, bevorzugt bei 78 bis 91 Gew.-%, insbesondere bei 80 bis 88,0 Gew.-% liegen. Alle Gewichtsangaben in Gew.-% beziehen sich herbei auf das in der Zubereitung (KM) enthaltene Gesamtgewicht an Wasser, die zum Gesamtgewicht der Zubereitung (KM) in Relation gesetzt wird.

Die kosmetische Zusammensetzung (KM) enthält als ersten wesentlichen Inhaltsstoff a) mindestens ein Oxidationsmittel. Bevorzugt werden hierbei bestimmte Oxidationsmittel eingesetzt. Es ist daher im Rahmen der vorliegenden Erfindung vorteilhaft, wenn die kosmetische Zusammensetzung (KM) mindestens eine oxidierende Verbindung, ausgewählt aus der Gruppe von Persulfaten, Chloriten, Wasserstoffperoxid und Anlagerungsprodukten von Wasserstoffperoxid an Harnstoff, Melamin sowie Natriumborat, insbesondere Wasserstoffperoxid, enthält. Der Einsatz von Wasserstoffperoxid hat sich erfindungsgemäß als besonders vorteilhaft erwiesen.

Die Konzentration des Oxidationsmittels in der Zusammensetzung (KM) wird einerseits von den gesetzlichen Vorgaben und andererseits von dem gewünschten Effekt bestimmt; vorzugsweise werden 0,5 bis 20,0 Gew.-%ige Lösungen in Wasser verwendet. Erfindungsgemäß bevorzugt ist es daher, wenn die kosmetische Zusammensetzung (KM) die mindestens eine oxidierende Verbindung, insbesondere Wasserstoffperoxid, in einer Gesamtmenge von 0,5 bis 20 Gew.-%, vorzugweise von 1,0 bis 18 Gew.-%, bevorzugt von 1,2 bis 16 Gew.-%, insbesondere von 1,5 bis 15 Gew.-%, bezogen auf das Gesamtgewicht der kosmetischen Zusammensetzung (KM), enthält. Je höher der Gehalt an Oxidationsmittel, insbesondere Wasserstoffperoxid, in der Zusammensetzung (KM) ist, desto größer ist die bei einer anteiligen Zersetzung des Oxidationsmittels entstehende Gasmenge. Höher konzentrierte Oxidationsmittel-haltige Zubereitungen sind demnach viel schwieriger lagerstabil in einer Verpackung (VP) zu konfektionieren als geringer konzentrierte Zubereitungen. Die Menge des Wasserstoffperoxids bezieht sich hierbei auf 100 %iges Wasserstoffperoxid.

Im Zuge der zu dieser Erfindung führenden Arbeiten hat sich herausgestellt, dass das erfindungsgemäße Produkt sich insbesondere auch zur Konfektionierung und stabilen Lagerung von höher konzentrierten Wasserstoffperoxid-Zubereitungen (KM) eignet. So zeigten erfindungsgemäße Verpackungen (VP), die Zubereitungen (KM) mit 9 bis 12 Gew.-% Wasserstoffperoxid enthielten, auch nach mehrwöchiger Lagerung bei erhöhter Temperatur keine Volumenveränderungen (d.h. kein Aufblähen) und keine unplanmäßigen Öffnungen (d.h. die Verpackungen platzten nicht auf).

Die kosmetische Zusammensetzung (KM) enthält als zweiten wesentlichen Inhaltsstoff b) mindestens einen C₈-C₃₀-Alkohol. In diesem Zusammenhang haben sich Mischungen von linearen C₁₆-C₁₈-Alkoholen besonders bewährt. Derartige Mischungen führen in Kombination mit dem weiteren Merkmal c) der Zusammensetzung (KM) zu einer hervorragenden Stabilisierung des mindestens einen Oxidationsmittels, insbesondere des Wasserstoffperoxids. Es ist daher im Rahmen der vorliegenden Erfindung vorteilhaft, wenn die kosmetische Zusammensetzung (KM) mindestens einen C₁₀-C₃₀-Alkohol ausgewählt aus der Gruppe von linearen C₁₀-C₂₈-Alkoholen, linearen C₁₂-C₂₆-Alkoholen, linearen C₁₄-C₂₀-Alkoholen, linearen C₁₄-C₁₈-Alkoholen sowie Mischungen der vorgenannten Alkohole, insbesondere einer Mischung aus linearen C₁₆-C₁₈-Alkoholen, enthält. Im Rahmen der vorliegenden Erfindung hat sich die unter dem Namen Cetearylalkohol bekannte Mischung aus Cetylalkohol und Stearylalkohol, insbesondere einer Mischung aus 50 Gew.-% Cetylalkohol und 50 Gew.-% Stearyalkohol, bezogen auf das Gesamtgewicht der Mischung, als besonders vorteilhaft erwiesen.

Der mindestens eine C₈-C₃₀-Alkohol wird bevorzugt in bestimmten Mengenbereichen eingesetzt. Bevorzugte Ausführungsformen der vorliegenden Erfindung sind daher dadurch gekennzeichnet, dass die kosmetische Zusammensetzung (KM) den mindestens eine C₈-C₃₀-Alkohol, insbesondere eine Mischung aus linearen C₁₆-C₁₈-Alkoholen, in einer Gesamtmenge von 0,1 bis 10 Gew.-%, vorzugweise von 0,50 bis 6,5 Gew.-%, bevorzugt von 1,0 bis 6,0 Gew.-%, insbesondere von 1,5 bis 5,0 Gew.-%, bezogen auf das Gesamtgewicht der kosmetischen Zusammensetzung (KM), enthält. Der Einsatz der zuvor angeführten Gesamtmengen des mindestens einen C₈-C₃₀-Alkohols, insbesondere der Mischung aus linearen C₁₆-C₁₈-Alkoholen, führt in Kombination mit den weiteren Bestandteilen der kosmetischen Zusammensetzung (KM) zu einer besonders guten Stabilisierung des in dieser Zusammensetzung enthaltenen Oxidationsmittels, insbesondere des Wasserstoffperoxids.

Als dritten wesentlichen Bestandteil c) enthält die kosmetische Zusammensetzung (KM) mindestens ein nichtionisches Tensid. Unter dem Begriff "nichtionisches Tensid" werden erfindungsgemäß amphiphile (bifunktionelle) Verbindungen verstanden, welche mindestens einen hydrophoben und mindestens einen hydrophilen Molekülteil aufweisen. Der hydrophobe Rest ist bevorzugt eine Kohlenwasserstoffkette mit 8 bis 28 Kohlenstoffatomen, die gesättigt oder ungesättigt, linear oder verzweigt sein kann. Besonders bevorzugt ist diese C₈-C₂₈-Alkylkette linear. Im Gegensatz zu anionischen, kationischen, zwitterionischen und amphiphilen Tensiden enthalten nichtionische Tenside jedoch weder kationische noch anionische Gruppen. Darüber hinaus weisen diese Tenside auch keinerlei kationisierbare und anionisierbare Gruppen auf, welche in Abhängigkeit vom pH-Wert kationische oder anionische Gruppierungen ausbilden können.

Durch die Kombination aus Fettalkohol und nichtionischem Tensid wird eine hervorragende Dispergierung der Bestandteile der kosmetischen Zusammensetzung (KM) und somit eine hohe Lagerstabilität erreicht. Zudem führt der Einsatz einer derartigen Kominationen zu einer guten Verteilbarkeit, insbesondere Vermischbarkeit, der kosmetischen Zusammensetzung (KM) mit der Zubereitung (B), welche die Oxidationsfarbstoffvorprodukte enthält. Es ist daher im Rahmen der vorliegenden Erfindung bevorzugt, wenn die kosmetische Zusammensetzung (KM) mindestens ein nichtionisches Tensid, ausgewählt aus der Gruppe von (i) ethoxylierten und/oder propoxylierten Alkoholen und Carbonsäuren mit 8 bis 30 Kohlenstoffatomen und 2 bis 30 Ethylenoxid- und/oder Propylenoxideinheiten pro Mol Alkohol, (ii) Anlagerungsprodukten von 30 bis 50 Mol Ethylenoxid an Rizinusöl und hydriertes Rizinusöl, (iii) Alkylpolyglucosiden der Formel R¹O-[G]ₚ, in welcher R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht, (iv) Monoethanolamiden von Carbonsäuren mit 8 bis 30 Kohlenstoffatomen sowie (v) deren Mischungen, insbesondere Anlagerungsprodukte von 40 Mol Ethylenoxid an hydriertes Rizinusöl, enthält. In der Formel R¹O-[G]ₚ gibt die Indexzahl p gibt den Oligomerisierungsgrad (DP), d. h. die Verteilung von Mono- und Oligoglucosiden, an und steht für eine Zahl zwischen 1 und 10. Während p in einer gegebenen Verbindung stets ganzzahlig sein muss und hier vor allem die Werte p = 1 bis 6 annehmen kann, ist der Wert p für ein bestimmtes Alkyloligoglucosid eine analytisch ermittelte rechnerische Größe, die meistens eine gebrochene Zahl darstellt. Erfindungsgemäß bevorzugt werden Alkyl- und/oder Alkenyloligoglucoside mit einem mittleren Oligomerisierungsgrad p von 1,1 bis 3,0 eingesetzt. Aus anwendungstechnischer Sicht sind solche Alkyl- und/oder Alkenyloligoglucoside bevorzugt, deren Oligomerisierungsgrad kleiner als 1,7 ist und insbesondere zwischen 1,2 und 1,7 liegt. Der Alkyl- bzw. Alkenylrest R¹ kann sich von primären Alkoholen mit 4 bis 20, vorzugsweise 8 bis 16 Kohlenstoffatomen ableiten. Erfindungsgemäß ganz besonders bevorzugt sind Alkyloligoglucoside auf der Basis von gehärtetem C_{12/14}-Kokosalkohol mit einem DP von 1-3, wie sie beispielsweise unter der INCI-Bezeichnung "Coco-Glucoside" im Handel erhältlich sind. Im Rahmen der vorliegenden Erfindung besonders bevorzugt eingesetzte nichtionische Tenside sind Anlagerungsprodukte von 40 Mol Ethylenoxid an hydriertes Rizinusöl, insbesondere die unter der INCI-Bezeichnung PEG-40 Hydrogenated Castor Oil (CAS-Nr.: 61788-85-0) bekannte Verbindung.

Um eine ausreichende Dispergierung aller Inhaltsstoffe des kosmetischen Mittels (KM) sicherzustellen, wird das mindestens eine nichtionische Tensid bevorzugt in bestimmten Gesamtmengen eingesetzt. Bevorzugte Ausführungsformen der vorliegenden Erfindung sind daher dadurch gekennzeichnet, dass die kosmetische Zusammensetzung (KM) das mindestens eine nichtionische Tensid, insbesondere die Mischung aus linearen C₁₆-C₁₈-Alkoholen, die mit durchschnittlich 20 Ethoxygruppen substituiert sind, in einer Gesamtmenge von 0,10 bis 5 Gew.-%, vorzugweise von 0,12 bis 3,5 Gew.-%, bevorzugt von 0,15 bis 1,8 Gew.-%, insbesondere von 0,50 bis 1,5 Gew.-%, bezogen auf das Gesamtgewicht der kosmetischen Zusammensetzung (KM), enthält.

Die kosmetische Zusammensetzung (KM) kann als einen weiteren Bestandteil mindestens ein flüssiges kosmetisches Öl umfassen. Unter dem Begriff "flüssige kosmetische Öle" werden im Rahmen der vorliegenden Erfindung für die kosmetische Verwendung geeignete Öle verstanden, welche in Wasser bei 20 °C unlöslich sind, sich also bevorzugt weniger als 1 Gew.-% des Öls, bezogen auf die Gesamtmenge der Wasser-Öl-Mischung, bei 20 °C in Wasser lösen. Bei dem erfindungsgemäß verwendeten kosmetischen Öl handelt es sich jedoch weder um Riechstoffe, noch um ätherische Öle. Bevorzugt werden im Rahmen der vorliegenden Erfindung Paraffinöle als kosmetische Öle eingesetzt.-Der Einsatz von kosmetischen Ölen führt zu einer erhöhten Stabilisierung des Oxidationsmittels, insbesondere des Wasserstoffperoxids, da dieses bei Dispergierung bzw. Emulgierung mit dem kosmetischen Öl von diesem umgeben und somit vor Zersetzung infolge der Reaktion mit weiteren Bestandteilen der kosmetischen Zusammensetzung (KM) geschützt wird. Im Rahmen der vorliegenden Erfindung werden bevorzugt bestimmte kosmetische Öle eingesetzt. Es ist daher erfindungsgemäß vorteilhaft, wenn die kosmetische Zusammensetzung (KM) mindestens ein flüssiges kosmetisches Öl, ausgewählt aus der Gruppe von (i) Estern von linearen oder verzweigten gesättigten oder ungesättigten C₂-C₃₀-Fettalkoholen mit linearen oder verzweigten gesättigten oder ungesättigten C₂-C₃₀-Fettsäuren, welche hydroxyliert sein können, (ii) C₈-C₂₂-Fettalkoholestern einwertiger oder mehrwertiger C₂-C₇-Hydroxycarbonsäuren, den Triethylcitraten, (iii) Mono-, Di- und Triglyceriden von linearen oder verzweigten, gesättigten oder ungesättigten, gegebenenfalls hydroxylierten C₈-C₃₀-Fettsäuren, (iv) Dicarbonsäureestern von linearen oder verzweigten C₂-C₁₀-Alkanolen, (v) symmetrischen, unsymmetrischen oder cyclischen Estern der Kohlensäure mit Fettalkoholen, den Estern von Dimeren ungesättigter C₁₂-C₂₂-Fettsäuren mit einwertigen, linearen, verzweigten und cyclischen C₂-C₁₈-Alkanolen oder C₂-C₆-Alkanolen, (vi) Benzoesäureestern von linearen oder verzweigten C₈-C₂₂-Alkanolen, wie Benzoesäure-C₁₂-C₁₅-Alkylester, Benzoesäureisostearylester und Benzoesäureoctyldodecylester, (vii) synthetischen Kohlenwasserstoffen, wie Polyisobuten und Polydecene, (viii) Kohlenwasserstoffen, wie Paraffinen, sowie (ix) deren Mischungen, insbesondere Paraffinöl, enthält. Im Rahmen der vorliegenden Erfindung hat es sich als besonders vorteilhaft herausgestellt, wenn als flüssiges kosmetisches Öl Paraffinöl, insbesondere die unter der INCI-Bezeichnung Paraffinum Liquidqum (CAS-Nr.: 8042-47-5) bekannte Verbindung, eingesetzt wird. Erfindungsgemäß bevorzugte Paraffinöle weisen dynamische Viskositäten bei 20 °C von 20 bis 150 mPa*s (gemessen gemäß DIN 51562-1 von 1999) auf.

Das mindestens eine flüssige kosmetische Öl, insbesondere das mindestens eine Paraffinöl, ist in den kosmetischen Zusammensetzungen (KM) bevorzugt in bestimmten Gesamtmengen enthalten. Hierdurch wird eine ausreichende Stabilisierung des Oxidationsmittels, insbesondere des Wasserstoffperoxids, erreicht. Bevorzugte Ausführungsformen der vorliegenden Erfindung sind daher dadurch gekennzeichnet, dass die kosmetische Zusammensetzung (KM) das mindestens eine flüssige kosmetische Öl, insbesondere Paraffinöl, in einer Gesamtmenge von 0,10 bis 25 Gew.-%, vorzugweise von 2,0 bis 24 Gew.-%, bevorzugt von 4,0 bis 22 Gew.-%, insbesondere von 5,0 bis 20 Gew.-%, bezogen auf das Gesamtgewicht der kosmetischen Zusammensetzung (KM), enthält.

Im Zuge der zu dieser Erfindung führenden Arbeiten hat sich herausgestellt, dass der Einsatz der zuvor genannten wesentlichen Inhaltsstoffe b) und c) gewährleistet, dass die kosmetische Zusammensetzung (KM), welche mindestens ein Oxidationsmittel enthält, in der speziellen Verpackung (VP) konfektioniert und gelagert werden kann, ohne dass sich diese Verpackung - welche eine Barriereschicht mit einer Durchtrittsperrwirkung für Gase und Wasserdampf besitzt - aufbläht oder platzt.

In diesem Zusammenhang hat sich eine ganz bestimmte Kombination der Inhaltsstoffe a) bis c) der kosmetischen Zusammensetzung (KM) als vorteilhaft herausgestellt. In einer bevorzugten Ausführungsform ist das erfindungsgemäße Produkt daher dadurch gekennzeichnet, dass die kosmetische Zusammensetzung (KM) Wasserstoffperoxid, eine Mischung aus linearen C₁₆-C₁₈-Alkoholen und als nichtionisches Tensid eine Mischung aus linearen C₁₆-C₁₈-Alkoholen, die mit durchschnittlich 20 Ethoxygruppen substituiert sind.

Zur weiteren Optimierung der Lagerstabilität werden die zuvor genannten Verbindungen bevorzugt in bestimmten Mengenbereichen in der Zubereitung (KM) eingesetzt. Besonders bevorzugte Ausführungsformen sind daher dadurch gekennzeichnet, dass die kosmetische Zusammensetzung (KM)
a) 1,5 bis 15 Gew.-% Wasserstoffperoxid,
b) 1 bis 5 Gew.-% einer Mischung aus linearen C₁₆-C₁₈-Alkoholen und
c) 0,5 bis 3,5 Gew.-% einer Mischung aus linearen C₁₆-C₁₈-Alkoholen, die mit durchschnittlich 20 Ethoxygruppen substituiert sind, jeweils bezogen auf das Gesamtgewicht des kosmetischen Mittels (KM), enthält.

Die kosmetische Zusammensetzung der vorliegenden Erfindung kann zusätzlich mindestens ein oder mehrere kationische Tenside enthalten. Unter dem Begriff "kationisches Tensid" sind erfindungsgemäß amphiphile (bifunktionelle) Verbindungen, welche aus mindestens einem hydrophoben und mindestens einem hydrophilen Molekülteil bestehen, zu verstehen. Der hydrophobe Rest ist bevorzugt eine Kohlenwasserstoffkette mit 8 bis 28 Kohlenstoffatomen, die gesättigt oder ungesättigt, linear oder verzweigt sein kann. Besonders bevorzugt ist diese C₈-C₂₈-Alkylkette linear. Zudem enthalten diese Tenside mindestens eine kationische Gruppe, insbesondere eine Ammonium- oder Alkylammoniumgruppe. Bevorzugt werden quaternäre Alkylammoniumverbindungen, insbesondere Tetraalkylammoiumverbindungen mit mindestens einem C₁₂- bis C₂₀-Alkylrest, mit einem physiologisch verträglichen Anion, insbesondere einem Chlorid, verwendet.

Typische Beispiele für diese Inhaltsstoffe sind Cetyl Trimethyl Ammonium Chlorid, Steartrimonium Chlorid, Behentrimonium Chlorid, Stearamidopropyl Trimonium Chlorid, Dioleylethyl Dimethyl Ammonium Methosulfat und Dioleylethyl Hydroxyethylmonium Methosulfat, insbesondere Steartrimonium Chlorid

Das mindestens eine kationische Tensid, insbesondere das Steartrimonium Chlorid, ist in der kosmetischen Zusammensetzung (KM) bevorzugt in bestimmten Gesamtmengen enthalten. Hierdurch wird sichergestellt, dass das anwendungsbereite Färbemittel stabil dispergiert auf das Haar aufgetragen werden kann. Bevorzugte Ausführungsformen der vorliegenden Erfindung sind daher dadurch gekennzeichnet, dass die kosmetische Zusammensetzung (KM) das mindestens eine kationische Tensid in einer Menge von 0,01 bis 2 Gew.-%, bevorzugt in einer Menge von 0,05 bis 1 Gew.-%, bevorzugter in einer Menge von 0,1 bis 0,5 Gew.-%, jeweils bezogen auf das Gesamtgewicht der kosmetischen Zusammensetzung, enthält.

Die kosmetische Zusammensetzung (KM) weist bevorzugt einen sauren pH-Wert auf, um eine Zersetzung des eingesetzten Oxidationsmittels, insbesondere des Wasserstoffperoxids, zu vermeiden bzw. zu vermindern. Es ist daher im Rahmen der vorliegenden Erfindung bevorzugt, wenn die kosmetische Zusammensetzung (KM) einen pH-Wert (gemessen bei 20 °C) von pH 1,5 bis pH 5,0, vorzugsweise von pH 2,0 bis pH 4,7, bevorzugt von pH 2,3 bis pH 4,4, insbesondere von pH 2,5 bis pH 4, aufweist.

Die in der Verpackung (VP) befindliche Zubereitung (KM) enthält die wesentlichen Inhaltsstoffe in einem wässrigen oder wässrig-alkoholischen Träger, bei dem es sich beispielsweise um eine Creme, eine Emulsion, ein Gel oder auch eine tensidhaltige schäumende Lösung handeln kann. Um die gewünschten Eigenschaften dieser Darreichunsformenen einzustellen, kann die Zubereitung (KM) weiterhin zusätzliche Wirk-, Hilfs- und Zusatzstoffe enthalten.

Die Zubereitung (KM) kann beispielsweise zusätzlich auch noch ein oder mehrere Säuren zur Stabilisierung des eingesetzten Oxidationsmittels, insbesondere des Wasserstoffperoxids, enthalten. Es ist daher im Rahmen der vorliegenden Erfindung bevorzugt, wenn die kosmetische Zusammensetzung (KM) zusätzlich mindestens eine Säure, ausgewählt aus der Gruppe von Dipicolinsäure, Zitronensäure, Essigsäure, Apfelsäure, Milchsäure, Weinsäure, Salzsäure, Phosphorsäure, Pyrophosphorsäure und deren Salzen, Benzoesäure sowie deren Salzen, 1-Hydroxyethan-1,1-diphosphonsäure, Ethylendiamintetraessigsäure und deren Salzen, Schwefelsäure sowie Mischungen, insbesondere ein Mischung aus Dipicolinsäure, Dinatriumpyrophosphat, Benzoesäure sowie deren Salze und 1-Hydroxyethan-1,1-diphosphonsäure, enthält.

Eine besonders hohe Stabilisierung des Oxidationsmittels, insbesondere des Wasserstoffperoxids, wird erreicht, wenn die zuvor genannten Säuren in bestimmten Mengenbereichen eingesetzt werden. Es ist daher in diesem Zusammenhang vorteilhaft, wenn die mindestens eine Säure, insbesondere die Mischung aus Dipicolinsäure, Dinatriumpyrophosphat, Benzoesäure sowie deren Salzen und 1-Hydroxyethan-1,1-diphosphonsäure, in einer Gesamtmenge von 0,1 bis 3,0 Gew.-%, vorzugsweise von 0,5 bis 2,5 Gew.-%, bevorzugt von 0,8 bis 2,0 Gew.-%, insbesondere von 0,9 bis 1,5 Gew.-%, bezogen auf das Gesamtgewicht der kosmetischen Zusammensetzung (KM), enthalten ist.

In nachfolgenden Tabellen sind besonders bevorzugte Ausführungsformen AF 1 bis AF 32 der in der Verpackung (VP) enthaltenen kosmetischen Zusammensetzung (KM) aufgeführt (alle Angaben in Gew.-%, sofern nichts anderes angegeben ist).

| | AF 1 | AF 2 | AF 3 | AF 4 |
|---|---|---|---|---|
| Oxidationsmittel | 0,5 - 20 | 1,0 - 18 | 1,2 - 16 | 1,5 - 15 |
| C₈-C₃₀-Alkohol | 0,10 - 7,0 | 0,50 - 6,5 | 1,0 - 6,0 | 1,5 - 5,0 |
| Nichtionisches Tensid | 0,10 - 2,5 | 0,12 - 2,0 | 0,15 - 1,8 | 0,30 - 1,5 |
| Kosmetischer Träger ¹⁾ | ad 100 | ad 100 | ad 100 | ad 100 |

| | AF 5 | AF 6 | AF 7 | AF 8 |
|---|---|---|---|---|
| Oxidationsmittel ²⁾ | 0,5 - 20 | 1,0 - 18 | 1,2 - 16 | 1,5 - 15 |
| C₈-C₃₀-Alkohol | 0,10 - 7,0 | 0,50 - 6,5 | 1,0 - 6,0 | 1,5 - 5,0 |
| Nichtionisches Tensid | 0,10 - 2,5 | 0,12 - 2,0 | 0,15 - 1,8 | 0,30 - 1,5 |
| Kosmetischer Träger ¹⁾ | ad 100 | ad 100 | ad 100 | ad 100 |

| | AF 9 | AF 10 | AF 11 | AF 12 |
|---|---|---|---|---|
| Oxidationsmittel ²⁾ | 0,5 - 20 | 1,0 - 18 | 1,2 - 16 | 1,5 - 15 |
| C₈-C₃₀-Alkohol ³⁾ | 0,10 - 7,0 | 0,50 - 6,5 | 1,0 - 6,0 | 1,5 - 5,0 |
| Nichtionisches Tensid | 0,10 - 2,5 | 0,12 - 2,0 | 0,15 - 1,8 | 0,30 - 1,5 |
| Kosmetischer Träger ¹⁾ | ad 100 | ad 100 | ad 100 | ad 100 |

| | AF 13 | AF 14 | AF 15 | AF 16 |
|---|---|---|---|---|
| Oxidationsmittel ²⁾ | 0,5 - 20 | 1,0 - 18 | 1,2 - 16 | 1,5 - 15 |
| C₈-C₃₀-Alkohol ³⁾ | 0,10 - 7,0 | 0,50 - 6,5 | 1,0 - 6,0 | 1,5 - 5,0 |
| Nichtionisches Tensid | 0,10 - 2,5 | 0,12 - 2,0 | 0,15 - 1,8 | 0,30 - 1,5 |
| Flüssiges kosmetisches Öl | 0,10 - 25 | 2,0 - 24 | 4,0 - 22 | 5,0 - 20 |
| Kosmetischer Träger ¹⁾ | ad 100 | ad 100 | ad 100 | ad 100 |

| | AF 17 | AF 18 | AF 19 | AF 20 |
|---|---|---|---|---|
| Oxidationsmittel ²⁾ | 0,5 - 20 | 1,0 - 18 | 1,2 - 16 | 1,5 - 15 |
| C₈-C₃₀-Alkohol ³⁾ | 0,10 - 7,0 | 0,50 - 6,5 | 1,0 - 6,0 | 1,5 - 5,0 |
| Nichtionisches Tensid ⁵⁾ | 0,10 - 2,5 | 0,12 - 2,0 | 0,15 - 1,8 | 0,30 - 1,5 |
| Flüssiges kosmetisches Öl | 0,10 - 25 | 2,0 - 24 | 4,0 - 22 | 5,0 - 20 |
| Kosmetischer Träger ¹⁾ | ad 100 | ad 100 | ad 100 | ad 100 |

| | AF 21 | AF 22 | AF 23 | AF 24 |
|---|---|---|---|---|
| Oxidationsmittel ²⁾ | 0,5 - 20 | 1,0 - 18 | 1,2 - 16 | 1,5 - 15 |
| C₈-C₃₀-Alkohol ³⁾ | 0,10 - 7,0 | 0,50 - 6,5 | 1,0 - 6,0 | 1,5 - 5,0 |
| Nichtionisches Tensid ⁵⁾ | 0,10 - 2,5 | 0,12 - 2,0 | 0,15 - 1,8 | 0,30 - 1,5 |
| Kosmetischer Träger ¹⁾ | ad 100 | ad 100 | ad 100 | ad 100 |

| | AF 25 | AF 26 | AF 27 | AF 28 |
|---|---|---|---|---|
| Oxidationsmittel ²⁾ | 0,5 - 20 | 1,0 - 18 | 1,2 - 16 | 1,5 - 15 |
| C₈-C₃₀-Alkohol ³⁾ | 0,10 - 7,0 | 0,50 - 6,5 | 1,0 - 6,0 | 1,5 - 5,0 |
| Kationisches Tensid ⁴⁾ | 0,10 - 3,0 | 0,12 - 2,5 | 0,15 - 2,0 | 0,20 - 1,5 |
| Nichtionisches Tensid ⁵⁾ | 0,10 - 2,5 | 0,12 - 2,0 | 0,15 - 1,8 | 0,30 - 1,5 |
| Flüssiges kosmetisches Öl ⁶⁾ | 0,10 - 25 | 2,0 - 24 | 4,0 - 22 | 5,0 - 20 |
| Säure | 0,1 - 3,0 | 0,5 - 2,5 | 0,8 - 2,0 | 0,9 - 1,5 |
| Kosmetischer Träger ¹⁾ | ad 100 | ad 100 | ad 100 | ad 100 |

| | AF 29 | AF 30 | AF 31 | AF 32 |
|---|---|---|---|---|
| Oxidationsmittel ²⁾ | 0,5 - 20 | 1,0 - 18 | 1,2 - 16 | 1,5 - 15 |
| C₈-C₃₀-Alkohol ³⁾ | 0,10 - 7,0 | 0,50 - 6,5 | 1,0 - 6,0 | 1,5 - 5,0 |
| Kationisches Tensid ⁴⁾ | 0,10 - 3,0 | 0,12 - 2,5 | 0,15 - 2,0 | 0,20 - 1,5 |
| Nichtionisches Tensid ⁵⁾ | 0,10 - 2,5 | 0,12 - 2,0 | 0,15 - 1,8 | 0,30 - 1,5 |
| Säure ⁷⁾ | 0,1 - 3,0 | 0,5 - 2,5 | 0,8 - 2,0 | 0,9 - 1,5 |
| Kosmetischer Träger ¹⁾ | ad 100 | ad 100 | ad 100 | ad 100 |

| | | | | |
|---|---|---|---|---|
| ¹⁾ wässriger oder wässrig-alkoholischer Träger ²⁾ Wasserstoffperoxid, Menge berechnet auf 100%-iges Wasserstoffperoxid, ³⁾ Mischung aus linearen C₁₆-C₁₈-Alkoholen, insbesondere Cetearylalkohol, ⁴⁾ Quarternäre Ammoniumverbindungen, insbesondere Steartrimonium Chlorid, ⁵⁾ eine Mischung aus linearen C₁₆-C₁₈-Alkoholen, die mit durchschnittlich 20 Ethoxygruppen substituiert sind, ⁶⁾ Paraffinöl, ⁷⁾ Mischung aus Dipicolinsäure, Dinatriumpyrophosphat, Benzoesäure sowie deren Salze und 1-Hydroxyethan-1,1-diphosphonsäure. | | | | |

Die zuvor beschriebenen Ausführungsformen AF 1 bis 32 werden jeweils in Verpackungen (VP) verpackt, welche die nachfolgend beschriebenen Anordnung der mehrlagigen Folie (F) aufweisen (von Innenraum (steht in Konakt mit der kosmetischen Zusammensetzung (KM)) zur Außenseite betrachtet):
a) *Innenraum* - Schicht (P1) - Schicht (P2) -Barriereschicht (BS) - *Außenseite*,
b) *Innenraum* - Schicht (P1) - Barriereschicht (BS) - Schicht (P2) - *Außenseite*,
c) *Innenraum* - Schicht (P2) - Schicht (P1) -Barriereschicht (BS) - *Außenseite*,
d) *Innenraum* - Schicht (P2) -Barriereschicht (BS) - Schicht (P1) - *Außenseite*,
e) *Innenraum* - Barriereschicht (BS) - Schicht (P1) -Schicht (P2) - *Außenseite*,
f) *Innenraum* - Barriereschicht (BS) - Schicht (P2) - Schicht (P1) - *Außenseite*,
g) *Innenraum* - Schicht (P1) - erste Klebeschicht (SK1) - Schicht (P2) - zweite Klebeschicht (SK2) - Barriereschicht (BS) - *Außenseite*,
h) *Innenraum* - Schicht (P1) - Klebeschicht (SK1) - Schicht (P2) - Barriereschicht (BS) - *Außenseite*,
i) *Innenraum* - Schicht (P1) - Schicht (P2) - zweite Klebeschicht (SK2) - Barriereschicht (BS) - *Außenseite*,
j) *Innenraum* - Barriereschicht (BS) - erste Klebeschicht (SK1) - Schicht (P1) - zweite Klebeschicht (SK2) - Schicht (P2) - *Außenseite*,
k) *Innenraum* - Barriereschicht (BS) - Klebeschicht (SK) - Schicht (P1) - Schicht (P2) - *Außenseite*,
l) *Innenraum* - Barriereschicht (BS) - Schicht (S1) - Klebeschicht (SK) - Schicht (P2) - *Außenseite*,
m) *Innenraum* - Schicht (P1) - erste Klebeschicht (SK1) - Barriereschicht (BS) - zweite Klebeschicht (SK2) - Schicht (P2) - *Außenseite*,
n) *Innenraum* - Schicht (P1) - Klebeschicht (SK) - Barriereschicht (BS) - Schicht (P2) - *Außenseite*,
o) *Innenraum* - Schicht (P1) - Barriere Schicht (BS) - Klebeschicht (SK) - Schicht (P2) - *Außenseite*.

Die auf diese Weise erhältlichen erfindungsgemäßen Produkte weisen eine hohe Lagerstabilität sowie einen im akzeptablen Bereich liegenden Wasserverlust während der Lagerung auf. Es wurde keine Auflähung oder Delaminierung der Verpackung (VP) während der Lagerung dieser erfindungsgemäßen kosmetischen Produkte beobachtet.

Das erfindungsgemäße Produkt wird zum Zweck der oxidativen Farbänderung eingesetzt. Hierfür wird die in der Verpackung (VP) verpackte Zubereitung (KM), bei welcher es sich um die Oxidationsmittelzubereitung handelt, zur Herstellung des anwendungsbereiten Farbänderungsmittels mit mindestens einer weiteren Zubereitung (B) vermischt. Zur Verhinderung von Inkompatibilitäten bzw. zur Vermeidung einer vorzeitigen Reaktion sind die Zubereitungen (KM) und (B) getrennt voneinander konfektioniert.

Ein besonders bevorzugtes erfindungsgemäßes Produkt ist dadurch gekennzeichnet, dass es eine von der Zubereitung (KM) getrennt konfektionierte Zubereitung (B) umfasst, wobei die Zubereitung (B) mindestens eine Verbindung, ausgewähl aus Oxidationsfarbstoffvorprodukten, direktziehenden Farbstoffen, Alkalisierungsmitteln sowie deren Mischungen enthält. Bevorzugte Produkte der vorliegenden Erfindung sind daher dadurch gekennzeichnet, dass es zusätzlich mindestens eine zweite kosmetische Zusammensetzung (KM2) umfasst, welche mindestens eine Verbindung, ausgewählt aus Oxidationsfarbstoffvorprodukten, direktziehenden Farbstoffen, Alkalisierungsmitteln sowie deren Mischungen enthält und welche getrennt von der kosmetischen Zusammensetzung (KM) konfektioniert ist.

Wird eine oxidative Färbung erwünscht, enthält die Zubereitung (B) mindestens ein Oxidationsfarbstoffvorprodukt. Oxidationsfarbstoffvorprodukte können in Enwickler und Kuppler unterteilt werden, wobei die Entwickler aufgrund ihrer größeren Empfindlichkeit gegenüber Sauerstoff meist in Form ihrer physiologisch verträglichen Salze (z.B. in Form ihrer Hydrochloride, Hydrobromide, Hydrogensulfate oder Sulfate) eingesetzt werden. Kupplerkomponenten bilden im Rahmen der oxidativen Färbung allein keine signifikante Färbung aus, sondern benötigen stets die Gegenwart von Entwicklerkomponenten. Bevorzugt enthalten solche Mittel zumindest ein Oxidationsfarbstoffvorprodukt vom Entwickler-Typ und mindestens ein Oxidationsfarbstoffvorprodukt vom Kuppler-Typ. Besonders geeignete Oxidationsfarbstoffvorprodukte vom Entwickler-Typ sind dabei ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen.

Besonders geeignete Oxidationsfarbstoffvorprodukte vom Kuppler-Typ sind dabei ausgewählt aus der Gruppe, gebildet aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxy-ethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen.

Zusätzlich kann die Zubereitung (B) auch noch ein oder mehrere direktziehende Farbstoffe enthalten. Geeignete nichtionische direktziehende Farbstoffe können ausgewählt werden aus der Gruppe, HC Yellow 2, HC Yellow 4, HC Yellow 5, HC Yellow 6, HC Yellow 12, HC Orange 1, Disperse Orange 3, HC Red 1, HC Red 3, HC Red 7,HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 4-[(3-Hydroxypropyl)amino]-3-nitrophenol, 4-Nitro-o-phenylendiamin, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Geeignete anionische direktziehende Farbstoffe können ausgewählt werden aus der Gruppe aus Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau.

Geeigenete kationische direktziehende Farbstoffe sind kationische Triphenylmethanfarbstoffe, wie beispielsweise Basic Blue 7, Basic Blue 26, Basic Violet 2 und Basic Violet 14, aromatische Systeme, die mit einer quaternären Stickstoffgruppe substituiert sind, wie beispielsweise Basic Yellow 57, Basic Red 76, Basic Blue 99, Basic Brown 16 und Basic Brown 17, kationische Anthrachinonfarbstoffe, wie HC Blue 16 (Bluequat B) sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls geeignete kationische direktziehende Farbstoffe.

Färbeprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Daher ist es bevorzugt, wenn der pH-Wert des Mittels (B) zwischen 7 und 11, insbesondere zwischen 8 und 10,5, liegt. Bei den pH-Werten im Sinne der vorliegenden Erfindung handelt es sich um pH-Werte, die bei einer Temperatur von 22 °C gemessen wurden.

Die Zubereitung (B) kann mindestens ein Alkalisierungsmittel enthalten. Die zur Einstellung des bevorzugten pH-Wertes erfindungsgemäß verwendbaren Alkalisierungsmittel können aus der Gruppe, die gebildet wird aus Ammoniak, Alkanolaminen, basischen Aminosäuren, sowie anorganischen Alkalisierungsmitteln wie (Erd-)Alkalimetallhydroxiden, (Erd-)Alkalimetallmetasilikaten, (Erd-)Alkalimetallphosphaten und (Erd-)Alkalimetallhydrogenphosphaten, ausgewählt werden. Bevorzugte anorganische Alkalisierungsmittel sind Magnesiumcarbonat, Natriumhydroxid, Kaliumhydroxid, Natriumsilicat und Natriummetasilicat. Erfindungsgemäß einsetzbare, organische Alkalisierungsmittel werden bevorzugt ausgewählt aus Monoethanolamin, 2-Amino-2-methyl-propanol und Triethanolamin. Die als erfindungsgemäßes Alkalisierungsmittel einsetzbaren basischen Aminosäuren werden bevorzugt ausgewählt aus der Gruppe, die gebildet wird aus Arginin, Lysin, Ornithin und Histidin, besonders bevorzugt Arginin. Es hat sich aber im Rahmen der Untersuchungen zur vorliegenden Erfindung herausgestellt, dass weiterhin erfindungsgemäß bevorzugte Mittel dadurch gekennzeichnet sind, dass sie zusätzlich ein organisches Alkalisierungsmittel enthalten. Eine Ausführungsform des ersten Erfindungsgegenstands ist dadurch gekennzeichnet, dass das Mittel zusätzlich mindestens ein Alkalisierungsmittel enthält, welches ausgewählt ist aus der Gruppe, die gebildet wird aus Ammoniak, Alkanolaminen und basischen Aminosäuren, insbesondere aus Ammoniak, Monoethanolamin und Arginin oder seinen verträglichen Salzen.

Die Zubereitung (B) kann weiterhin zusätzliche Wirk-, Hilfs- und Zusatzstoffe enthalten. So können beispielsweise ein oder mehrere Fettbestandteile aus der Gruppe der C₁₂-C₃₀-Fettalkohole, der C₁₂-C₃₀-Fettsäuretriglyceride, der C₁₂-C₃₀-Fettsäuremonoglyceride, der C₁₂-C₃₀-Fettsäurediglyceride und/oder der Kohlenwasserstoffe enthalten sein.

Vorzugsweise kann in der Zubereitung (B) zusätzlich eine oberflächenaktive Substanz zugesetzt werden, wobei solche oberflächenaktive Substanzen je nach Anwendungsgebiet als Tenside oder als Emulgatoren bezeichnet werden: Sie sind bevorzugt aus anionischen, zwitterionischen, amphoteren und nichtionischen Tensiden und Emulgatoren ausgewählt.

Bevorzugt enthält die Zubereitung (B) mindestens ein anionisches Tensid. Bevorzugte anionische Tenside sind Fettsäuren, Alkylsulfate, Alkylethersulfate und Ethercarbonsäuren mit 10 bis 20 C-Atomen in der Alkylgruppe und bis zu 16 Glykolethergruppen im Molekül.

Weiterhin kann die Zubereitung (B) zusätzlich mindestens ein zwitterionisches Tensid enthalten. Bevorzugte zwitterionische Tenside sind Betaine, N-Alkyl-N,N-dimethylammonium-glycinate, N-Acyl-aminopropyl-N,N-dimethylammoniumglycinate, und 2-Alkyl-3-carboxymethyl-3-hydroxyethyl-imidazoline. Ein bevorzugtes zwitterionisches Tensid ist unter der INCI-Bezeichnung Cocamidopropyl Betaine bekannt.

Darüber hinaus kann es vorgesehen sein, dass die Zubereitung (B) mindestens ein amphoteres Tensid enthält. Bevorzugte amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside sind N-Kokosalkylaminopropionat, as Kokosacylaminoethylaminopropionat und C₁₂-C₁₈-Acylsarcosin.

Weiterhin hat es sich als vorteilhaft erwiesen, wenn die Zubereitung (B) weitere, nichtionogene grenzflächenaktive Stoffe, enthalten. Bevorzugte nichtionische Tenside sind Alkylpolyglykoside sowie Alkylenoxid-Anlagerungsprodukte an Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Die nichtionischen, zwitterionischen oder amphoteren Tenside werden in Anteilen von 0,1 bis 45 Gew.%, bevorzugt 1 bis 30 Gew.% und ganz besonders bevorzugt von 1 bis 15 Gew.%, bezogen auf die das Gesamtgewicht der Zubereitung (B), eingesetzt.

Die Zubereitung (B) kann zusätzlich auch mindestens ein Verdickungsmittel enthalten. Bezüglich dieser Verdickungsmittel bestehen keine prinzipiellen Einschränkungen. Es können sowohl organische als auch rein anorganische Verdickungsmittel zum Einsatz kommen. Geeignete Verdickungsmittel sind anionische, synthetische Polymere, kationische, synthetische Polymere, natürlich vorkommende Verdickungsmittel, wie nichtionische Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, sowie Cellulosederivate, wie beispielsweise Methylcellulose, Carboxyalkylcellulosen und Hydroxyalkylcellulosen, nichtionische, vollsynthetische Polymere, wie Polyvinylalkohol oder Polyvinylpyrrolidinon; sowie anorganische Verdickungsmittel, insbesondere Schichtsilikate wie beispielsweise Bentonit, besonders Smektite, wie Montmorillonit oder Hectorit.

Ferner kann die Zubereitung (B) weitere Wirk-, Hilfs- und Zusatzstoffe enthalten, wie beispielsweise nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidinon-Copolymere, Vinylpyrrolidinon-Imidazoliniummethochlorid-Copolymere und quaternierter Polyvinylalkohol; zwitterionische und amphotere Polymere; anionische Polymere wie beispielsweise Polyacrylsäuren oder vernetzte Polyacrylsäuren; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; Fettstoffe und pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat sowie Pigmente.

Die Auswahl dieser weiteren Stoffe wird der Fachmann gemäß der gewünschten Eigenschaften der Zubereitung (B) sowie des erfindungsgemäßen Produktes treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in der Zubereitung (B) bevorzugt in Mengen von jeweils 0,0001 bis 25 Gew.-%, insbesondere von 0,0005 bis 15 Gew.-%, jeweils bezogen auf das Gesamtgewicht der Zubereitung (B) eingesetzt.

Die folgenden Beispiele erläutern die vorliegende Erfindung, ohne sie jedoch darauf einzuschränken:

### Beispiele:

Auf eine Folienschicht aus Polyethylenterephthalat mit einer Dicke von 40 µm (Mikrometer) wurde eine 100 nm dicke Schicht aus Siliciumdioxid SiOx aufgedampft. Anschließend wurde die SiOx-Schicht mit ca. 3 g/m² ORMOCER-Polymer überlackiert und ausgehärtet. Auf die ORMOCER-Schicht wurde dann eine 70 µm (Mikrometer) dicke Schicht aus Polyethylen aufgebracht. Aus der Folie wurde eine Verpackung (VP) hergestellt.

Es wurden die folgenden kosmetischen Zusammensetzungen (KM) eingesetzt (alle Angaben in Gew.-%).

| **Inhaltsstoffe** | **KM** |
|---|---|
| Kaliumhydroxid (50%ig) | 0,19 |
| Natriumbenzoat | 0,04 |
| Dipicolinsäure | 0,10 |
| Dinatriumpyrophosphat | 0,10 |
| 1-Hydroxyethan-1,1-diphosphonsäure (60%ig) | 0,25 |
| Propandiol-1,2 | 1,0 |
| Oxidationsmittel ¹⁾ | 12 |
| C₈-C₃₀-Alkohol ²⁾ | 3,40 |
| Kationisches Tensid ³⁾ | 0,39 |
| Nichtionisches Tensid ⁴⁾ | 1,0 |
| Flüssiges kosmetisches Öl ⁵⁾ | 0,3 |
| Wasser | ad 100 |

| | |
|---|---|
| ¹⁾ bevorzugt Wasserstoffperoxid, berechnet auf 100%iges H₂O₂, ²⁾ bevorzugt eine Mischung aus linearen C₁₄-C₁₈-Alkoholen, insbesondere Cetearylalkohol, ³⁾ bevorzugt ein Alkylammoniumsalz, insbesondere Steartrimonium Chloride, ⁴⁾ bevorzugt ein Anlagerungsprodukte von 40 Mol Ethylenoxid an hydriertes Rizinusöl, insbesondere PEG-40 hydrogenated Castor Oil; oder Ceteareth-20, ^{2,3,4)} die Inhaltsstoffe können auch als ein kommerziell erhältliches Rohprodukt (Emulgade^{®} F) in der beispielhaften kosmetischen Zusammensetzung enthalten sein (4,5 Gew.-%). ⁵⁾ bevorzugt Paraffinöl | |

| **Inhaltsstoffe** | **KM** |
|---|---|
| Kaliumhydroxid (50%ig) | 0,19 |
| Natriumbenzoat | 0,04 |
| Dipicolinsäure | 0,10 |
| Dinatriumpyrophosphat | 0,10 |
| 1-Hydroxyethan-1,1-diphosphonsäure (60%ig) | 0,25 |
| Oxidationsmittel ¹⁾ | 9,1 |
| C₈-C₃₀-Alkohol ²⁾ | 3,40 |
| Kationisches Tensid ³⁾ | 0,39 |
| Nichtionisches Tensid ⁴⁾ | 1,0 |
| Flüssiges kosmetisches Öl ⁵⁾ | 0,3 |
| Propandiol-1,2 | 1,0 |
| Wasser | ad 100 |

| | |
|---|---|
| ¹⁾ bevorzugt Wasserstoffperoxid, berechnet auf 100%iges H₂O₂, ²⁾ bevorzugt eine Mischung aus linearen C₁₄-C₁₈-Alkoholen, insbesondere Cetearylalkohol, ³⁾ bevorzugt ein Alkylammoniumsalz, insbesondere Steartrimonium Chloride, ⁴⁾ bevorzugt ein Anlagerungsprodukte von 40 Mol Ethylenoxid an hydriertes Rizinusöl, insbesondere PEG-40 hydrogenated Castor Oil; oder Ceteareth-20, ⁵⁾ bevorzugt Paraffinöl | |

Die kosmetisch Zusammensetzung KM in die zuvor beschriebenen Verpackungen (VP) abgefüllt. Dann wurden die Verpackungen bei 24 Wochen bei 40 °C gelagert. Die Verpackungen waren nicht aufgebläht oder delaminiert.

## Patentansprüche

1. Kosmetisches Produkt zur Veränderung der natürlichen Farbe keratinischer Fasern, insbesondere menschlicher Haare, umfassend
(i) mindestens eine Verpackung (VP), umfassend mindestens eine mehrlagige Folie (F), welche mindestens eine erste Polymerschicht (P1), mindestens eine zweite Polymerschicht (P2) sowie mindestens eine Barriereschicht (BS) enthält, und
(ii) mindestens eine kosmetische Zusammensetzung (KM), welche in der Verpackung (VP) verpackt ist und enthält:
a) mindestens eine oxidierende Verbindung,
b) mindestens einen C₈-C₃₀-Alkohol, und
c) mindestens ein nichtionisches Tensid,
**dadurch gekennzeichnet, dass** die erste Polymerschicht (P1) aus Polyethylenterephthalat gebildet ist; die zweite Polymerschicht (P2) aus Polyethylen gebildet ist; und die Barriereschicht (BS) aus einem mit einer SiOₓ-Schicht versehenen Polyethylenterephthalat gebildet ist.

2. Kosmetisches Produkt nach Anspruch 1, **dadurch gekennzeichnet, dass** die erste Polymerschicht (P1) eine Schichtdicke von 4 µm bis 50 µm, bevorzugt von 5 µm bis 35 µm, bevorzugter von 6 µm bis 20 µm, aufweist; die zweite Polymerschicht (P2) eine Schichtdicke von 20 µm bis 150 µm, bevorzugt von 30 µm bis 110 µm, bevorzugter von 40 µm bis 90 µm, aufweist; und/oder die Schichtdicke der Barriereschicht (BS) 4 µm bis 20 µm, bevorzugt von 5 µm bis 18 µm, bevorzugter von 6 µm bis 15 µm, beträgt.

3. Kosmetisches Produkt nach Anspruch 1 oder Anspruch 2, **dadurch gekennzeichnet, dass** die mehrlagige Folie (F) eine Sauerstoffdurchlässigkeitsrate (OTR) bei 23 °C und 50 % relativer Luftfeuchtigkeit von 0,1 bis 5 cc/m²/d/bar, bevorzugt von 0,2 bis 3,5 cc/m²/d/bar, bevorzugter von 0,5 bis 2,5 cc/m²/d/bar, und eine Wasserdampfdurchlässigkeit bei 38 °C und 100 % relativer Luftfeuchtigkeit von 0,1 bis 5 g/m²d, bevorzugt von 0,2 bis 3,5 g/m²d, bevorzugter von 0,5 bis 2,5 g/m²d, aufweist.

4. Kosmetisches Produkt nach einem der vorangehenden Ansprüche, **dadurch gekennzeichnet, dass** die Haftfestigkeit der Folie 0,1 bis 10 N/15mm, bevorzugt 1 bis 8 N/15mm, bevorzugter 1,5 bis 5 N/15mm, beträgt; und/oder **dadurch gekennzeichnet, dass** die Siegelnahtfestigkeit der Verpackung (VP) 10 bis 40 N/15mm, bevorzugt 15 bis 35 N/15mm, bevorzugter 20 bis 30 N/15mm, unter den Bedingungen 150 °C, 2,54 cm (1") und 4 kg/cm² beträgt.

5. Kosmetisches Produkt nach einem der vorangehenden Ansprüche, **dadurch gekennzeichnet, dass** die mindestens eine mehrlagige Folie die mindestens eine Barriereschicht (BS) zwischen der mindestens einen ersten Polymerschicht (P1) und der mindestens einen zweiten Polymerschicht (P2) enthält.

6. Kosmetisches Produkt nach einem der vorangehenden Ansprüche, **dadurch gekennzeichnet, dass** die erste Polymerschicht (P1) die Außenschicht bildet.

7. Kosmetisches Produkt nach einem der vorangehenden Ansprüche, **dadurch gekennzeichnet, dass** die kosmetische Zusammensetzung (KM) einen pH-Wert (gemessen bei 20 °C) von pH 1,5 bis pH 5,0, vorzugsweise von pH 2,0 bis pH 4,7, bevorzugt von pH 2,3 bis pH 4,4, insbesondere von pH 2,5 bis pH 4, aufweist.

8. Kosmetisches Produkt nach einem der vorangehenden Ansprüche, **dadurch gekennzeichnet, dass** die kosmetische Zusammensetzung (KM) die mindestens eine oxidierende Verbindung, insbesondere Wasserstoffperoxid, in einer Gesamtmenge von 0,5 bis 20 Gew.-%, vorzugweise von 1,0 bis 18 Gew.-%, bevorzugt von 1,2 bis 16 Gew.-%, insbesondere von 1,5 bis 15 Gew.-%, bezogen auf das Gesamtgewicht der kosmetischen Zusammensetzung (KM), enthält.

9. Kosmetisches Produkt nach einem der vorangehenden Ansprüche, **dadurch gekennzeichnet, dass** die kosmetische Zusammensetzung (KM) das mindestens eine nichtionische Tensid, insbesondere die Mischung aus linearen C₁₆-C₁₈-Alkoholen, die mit durchschnittlich 20 Ethoxygruppen substituiert sind, in einer Gesamtmenge von 0,1 bis 5 Gew.-%, vorzugweise von 0,12 bis 3,5 Gew.-%, bevorzugt von 0,15 bis 1,8 Gew.-%, insbesondere von 0,50 bis 1,5 Gew.-%, bezogen auf das Gesamtgewicht der kosmetischen Zusammensetzung (KM), enthält.

10. Kosmetisches Produkt nach einem der vorangehenden Ansprüche, **dadurch gekennzeichnet, dass** der C₈-C₃₀-Alkohol eine Mischung aus linearen C₁₆-C₁₈-Alkoholen ist, die in einer Gesamtmenge von 0,1 bis 10 Gew.-%, vorzugweise von 0,50 bis 6,5 Gew.-%, bevorzugt von 1,0 bis 6,0 Gew.-%, insbesondere von 1,5 bis 5,0 Gew.-%, bezogen auf das Gesamtgewicht der kosmetischen Zusammensetzung (KM), in der kosmetischen Zusammensetzung (KM) enthalten sind.

## Claims

1. A cosmetic product for changing the natural color of keratinous fibers, in particular human hair, the product comprising
(i) at least one packaging (VP) comprising at least one multilayer film (F) which contains at least one first polymer layer (P1), at least one second polymer layer (P2) and at least one barrier layer (BS), and
(ii) at least one cosmetic composition (KM) which is packaged in the packaging (VP) and contains:
a) at least one oxidizing compound,
b) at least one C₈-C₃₀ alcohol, and
c) at least one non-ionic surfactant,
**characterized in that** the first polymer layer (P1) is formed from polyethylene terephthalate; the second polymer layer (P2) is formed from polyethylene; and the barrier layer (BS) is formed from a polyethylene terephthalate provided with a SiOₓ layer.

2. The cosmetic product according to claim 1, **characterized in that** the first polymer layer (P1) has a layer thickness of from 4 µm to 50 µm, preferably from 5 µm to 35 µm, more preferably from 6 µm to 20 µm; the second polymer layer (P2) has a layer thickness of 20 µm to 150 µm, preferably from 30 µm to 110 µm, more preferably from 40 µm to 90 µm; and/or the layer thickness of the barrier layer (BS) is from 4 µm to 20 µm, preferably from 5 µm to 18 µm, more preferably from 6 µm to 15 µm.

3. The cosmetic product according to either claim 1 or claim 2,
**characterized in that** the multi-layer film (F) has an oxygen transmission rate (OTR) at 23 °C and 50% relative humidity of from 0.1 to 5 cc/m²/d/bar, preferably from 0.2 to 3.5 cc/m²/d/bar, more preferably from 0.5 to 2.5 cc/m²/d/bar, and a water vapor transmission at 38 °C and 100% relative humidity of from 0.1 to 5 g/m²d, preferably from 0.2 to 3.5 g/m²d, more preferably from 0.5 to 2.5 g/m²d.

4. The cosmetic product according to one of the preceding claims,
**characterized in that** the adhesive strength of the film is 0.1 to 10 N/15mm, preferably 1 to 8 N/15mm, more preferably 1.5 to 5 N/15mm; **and/or**
**characterized in that** the seal seam strength of the packaging (VP) is 10 to 40 N/15mm, preferably 15 to 35 N/15mm, more preferably 20 to 30 N/15mm, under the conditions 150 °C, 2.54 cm (1 ") and 4 kg/cm².

5. The cosmetic product according to one of the preceding claims,
**characterized in that** the at least one multilayer film contains the at least one barrier layer (BS) between the at least one first polymer layer (P1) and the at least one second polymer layer (P2).

6. The cosmetic product according to one of the preceding claims,
**characterized in that** the first polymer layer (P1) forms the outer layer.

7. The cosmetic product according to one of the preceding claims,
**characterized in that** the cosmetic composition (KM) has a pH (measured at 20 °C) of from pH 1.5 to pH 5.0, preferably from pH 2.0 to pH 4.7, more preferably from pH 2.3 to pH 4.4, in particular from pH 2.5 to pH 4.

8. The cosmetic product according to one of the preceding claims,
**characterized in that** the cosmetic composition (KM) contains the at least one oxidizing compound, in particular hydrogen peroxide, in a total amount of from 0.5 to 20 wt%, preferably from 1.0 to 18 wt%, more preferably from 1.2 to 16 wt%, in particular from 1.5 to 15 wt%, based on the total weight of the cosmetic composition (KM).

9. The cosmetic product according to one of the preceding claims,
**characterized in that** the cosmetic composition (KM) contains the at least one non-ionic surfactant, in particular the mixture of linear C₁₆-C₁₈ alcohols substituted with an average of 20 ethoxy groups, in a total amount of 0.1 to 5 wt.%, preferably 0.12 to 3.5 wt.%, more preferably 0.15 to 1.8 wt.%, in particular 0.50 to 1.5 wt.%, based on the total weight of the cosmetic composition (KM).

10. The cosmetic product according to one of the preceding claims,
**characterized in that** the C₈-C₃₀ alcohol is a mixture of linear C₁₆-C₁₈ alcohols which are contained in the cosmetic composition (KM) in a total amount of 0.1 to 10 wt.%, preferably 0.50 to 6.5 wt.%, preferably 1.0 to 6.0 wt.%, in particular 1.5 to 5.0 wt.%, based on the total weight of the cosmetic composition (KM).

## Revendications

1. Produit cosmétique permettant de modifier la couleur naturelle de fibres kératiniques, en particulier de cheveux humains, comprenant
(i) au moins un emballage (VP), comprenant au moins un film multicouche (F) qui contient au moins une première couche polymère (P1), au moins une seconde couche polymère (P2) et au moins une couche barrière (BS), et
(ii) au moins une composition cosmétique (KM) emballée dans l'emballage (VP) et contenant :
a) au moins un composé oxydant,
b) au moins un alcool en C₈-C₃₀, et
c) au moins un tensioactif non ionique,
**caractérisé en ce que** la première couche polymère (P1) est formée à partir de polyéthylène téréphtalate ; la seconde couche polymère (P2) est formée à partir de polyéthylène ; et la couche barrière (BS) est formée à partir d'un polyéthylène téréphtalate pourvu d'une couche de SiOₓ.

2. Produit cosmétique selon la revendication 1, **caractérisé en ce que** la première couche polymère (P1) présente une épaisseur de couche allant de 4 µm à 50 µm, de préférence de 5 µm à 35 µm, plus préférablement de 6 µm à 20 µm ; la seconde couche polymère (P2) présente une épaisseur de couche allant de 20 µm à 150 µm, de préférence de 30 µm à 110 µm, plus préférablement de 40 µm à 90 µm ; et/ou l'épaisseur de couche de la couche barrière (BS) va de 4 µm à 20 µm, de préférence de 5 µm à 18 µm, plus préférablement de 6 µm à 15 µm.

3. Produit cosmétique selon la revendication 1 ou la revendication 2,
**caractérisé en ce que** le film multicouche (F) présente un taux de transmission à l'oxygène (OTR) à 23 °C et à 50 % d'humidité de l'air relative allant de 0,1 à 5 cc/m²/d/bar, de préférence de 0,2 à 3,5 cc/m²/d/bar, plus préférablement de 0,5 à 2,5 cc/m²/d/bar, et une perméabilité à la vapeur d'eau à 38 °C et à 100 % d'humidité de l'air relative allant de 0,1 à 5 g/m²d, de préférence de 0,2 à 3,5 g/m²d, plus préférablement de 0,5 à 2,5 g/m²d.

4. Produit cosmétique selon l'une des revendications précédentes,
**caractérisé en ce que** l'adhésivité du film va de 0,1 à 10 N/15 mm, de préférence de 1 à 8 N/15 mm, plus préférablement de 1,5 à 5 N/15 mm ;
**et/ou caractérisé en ce que** la résistance de joint thermoscellé de l'emballage (VP) va de 10 à 40 N/15 mm, de préférence de 15 à 35 N/15 mm, plus préférablement de 20 à 30 N/15 mm, dans les conditions de 150 °C, 2,54 cm (1") et 4 kg/cm².

5. Produit cosmétique selon l'une des revendications précédentes,
**caractérisé en ce que** l'au moins un film multicouche contient l'au moins une couche barrière (BS) entre l'au moins une première couche polymère (P1) et l'au moins une seconde couche polymère (P2).

6. Produit cosmétique selon l'une des revendications précédentes,
**caractérisé en ce que** la première couche polymère (P1) forme la couche externe.

7. Produit cosmétique selon l'une des revendications précédentes,
**caractérisé en ce que** la composition cosmétique (KM) présente un pH (mesuré à 20 °C) allant de pH 1,5 à pH 5,0, préférentiellement de pH 2,0 à pH 4,7, de préférence de pH 2,3 à pH 4,4, en particulier de pH 2,5 à pH 4.

8. Produit cosmétique selon l'une des revendications précédentes,
**caractérisée en ce que** la composition cosmétique (KM) contient l'au moins un composé oxydant, en particulier de l'eau oxygénée, en une quantité totale allant de 0,5 à 20 % en poids, préférentiellement de 1,0 à 18 % en poids, de préférence de 1,2 à 16 % en poids, en particulier de 1,5 à 15 % en poids, par rapport au poids total de la composition cosmétique (KM).

9. Produit cosmétique selon l'une des revendications précédentes,
**caractérisé en ce que** la composition cosmétique (KM) contient l'au moins un tensioactif non ionique, en particulier le mélange d'alcools linéaires en C₁₆-C₁₈ substitués par en moyenne 20 groupes éthoxy, en une quantité totale allant de 0,1 à 5 % en poids, préférentiellement de 0,12 à 3,5 % en poids, de préférence de 0,15 à 1,8 % en poids, en particulier de 0,50 à 1,5 % en poids, par rapport au poids total de la composition cosmétique (KM).

10. Produit cosmétique selon l'une des revendications précédentes,
**caractérisé en ce que** l'alcool en C₈-C₃₀ est un mélange d'alcools linéaires en C₁₆-C₁₈ présents dans la composition cosmétique (KM) en une quantité totale allant de 0,1 à 10 % en poids, préférentiellement de 0,50 à 6,5 % en poids, de préférence de 1,0 à 6,0 % en poids, en particulier de 1,5 à 5,0 % en poids, par rapport au poids total de la composition cosmétique (KM).
